# EUROPEAN PATENT SPECIFICATION

(11) **EP 1 718 977 B1**
(45) Date of publication and mention of the grant of the patent: **10.12.2008**
(21) Application number: 04794025.9
(22) Date of filing: 04.10.2004
(51) Int. Cl.: G01N 33/68, C12Q 1/48, C07K 16/00, C07K 7/00

(54) **PROTEIN PHOSPHORYLATION IN T-CELL RECEPTOR SIGNALING PATHWAYS**
PROTEINPHOSPHORYLIERUNG IN T-ZELLREZEPTOR-SIGNALWEGEN
PHOSPHORYLATION DE PROTEINES DANS LES VOIES DE SIGNALISATION DU RECEPTEUR DE LYMPHOCYTES T

(30) Priority: 12.02.2004 US 777893
(43) Date of publication of application: 08.11.2006
(73) Proprietor: Cell Signaling Technology, Inc., Danvers, MA 01923 (US)
(72) Inventor: MORITZ, Albrecht, Salem, MA 01970 (US); LEE, Kimberly, Boston, MA 02114 (US); RUSH, John, Beverly, MA 01915 (US); POLAKIEWICZ, Roberto, Lexington, MA 02421 (US)
(74) Representative: Hill, Justin John
(86) International application number: PCT/US2004/032511
(87) International publication number: WO 2005/083444

(56) References cited:
- WO-A-03/016861
- US-A1- 2003 044 848
- LEE K-H ET AL: "T cell receptor signaling precedes immunological synapse formation" SCIENCE, vol. 295, no. 5559, 22 February 2002 (2002-02-22), pages 1539-1542, XP002322781
- MUSTELIN T ET AL: "Positive and negative regulation of T-cell activation through kinases and phosphatases" BIOCHEMICAL JOURNAL, vol. 371, no. 1, 1 April 2003 (2003-04-01), pages 15-27, XP002322737
- CELL SIGNALING TECHNOLOGY: "Phospho-LAT (Tyr191) Antibody" INTERNET CITATION, [Online] May 2002 (2002-05), XP002322738 Retrieved from the Internet: URL:http://www.cellsignal.com/pdf/3584.pdf > [retrieved on 2005-03-31]
- BD BIOSCIENCES: "BD(TM) Phospho-Specific Solutions" INTERNET CITATION, [Online] 2003, XP002322739 Retrieved from the Internet: URL:http://www.bdbeurope.com/temp/457849.p df> [retrieved on 2005-03-31]
- IAVARONE A ET AL: "Repression of the CDK activator Cdc25A and cell-cycle arrest by cytokine TGF-beta in cells lacking the CDK inhibitor p15." NATURE, vol. 387, no. 6631, 22 May 1997 (1997-05-22), pages 417-422, XP002447040
- LUCAS J J ET AL: "Regulation of synthesis and activity of the PLSTIRE protein (cyclin-dependent kinase 6 (cdk6)), a major cyclin D-associated cdk4 homologue in normal human T lymphocytes." JOURNAL OF IMMUNOLOGY, vol. 154, no. 12, 15 June 1995 (1995-06-15), pages 6275-6284, XP002447041
- NAGASAWA M ET AL: "Rapid nuclear translocation and increased activity of cyclin-dependent kinase 6 after T cell activation." JOURNAL OF IMMUNOLOGY, vol. 158, no. 11, 1 June 1997 (1997-06-01), pages 5146-5154, XP002447042
- MEYERSON M ET AL: "A family of human cdc2-related protein kinases" EMBO JOURNAL, vol. 11, no. 8, 1992, pages 2909-2917, XP002128265 -& DATABASE UniProt [Online] 1 April 1993 (1993-04-01), "Cell division protein kinase 6 (EC 2.7.11.22) (Serine/threonine- protein kinase PLSTIRE)." XP002447044 retrieved from EBI accession no. UNIPROT:Q00534 Database accession no. Q00534
- RUSH J ET AL: "Immunoaffinity profiling of tyrosine phosphorylation in cancer cells" NATURE BIOTECHNOLOGY, vol. 23, no. 1, January 2005 (2005-01), pages 94-101, XP002322741

## Description

### FIELD OF THE INVENTION

The invention relates generally to antibodies and peptide reagents for the detection of protein phosphorylation, and to protein phosphorylation in cancer.

### BACKGROUND OF THE INVENTION

The activation of proteins by post-translational modification represents an important cellular mechanism for regulating most aspects of biological organization and control, including growth, development, homeostasis, and cellular communication. For example, protein phosphorylation plays a critical role in the etiology of many pathological conditions and diseases, including cancer, developmental disorders, autoimmune diseases, and diabetes, as well as in proper immune function. In spite of the importance of protein modification, it is not yet well understood at the molecular level. The reasons for this lack of understanding are, first, that the cellular modification system is extraordinarily complex, and second, that the technology necessary to unravel its complexity has not yet been fully developed.

The complexity of protein modification, including phosphorylation, on a proteorne-wide scale derives from three factors: the large number of modifying proteins, e.g. kinases, encoded in the genome, the much larger number of sites on substrate proteins that are modified by these enzymes, and the dynamic nature of protein expression during growth, development, disease states, and aging. The human genome encodes, for example, over 520 different protein kinases, making them the most abundant class of enzymes known. See Hunter, Nature 411: 355-65 (2001). Each of these kinases phosphorylates specific serine, threonine, or tyrosine residues located within distinct amino acid sequences, or motifs, contained within different protein substrates. Most kinases phosphorylate many different proteins: it is estimated that one-third of all proteins encoded by the human genome are phosphorylated, and many are phosphorylated at multiple sites by different kinases. *See* Graves et al., Pharmacol. Ther. 82: 111-21 (1999).

Many of these phosphorylation sites regulate critical biological processes and may prove to be important diagnostic or therapeutic targets for molecular medicine. For example, of the more than 100 dominant oncogenes identified to date, 46 are protein kinases. See Hunter, *supra.* Oncogenic kinases such as ErbB2 and Jak3, widely expressed in breast tumors and various leukemias, respectively, transform cells to the oncogenic phenotype at least in part because of their ability to phosphorylate cellular proteins. Understanding which proteins are modified by these kinases will greatly expand our understanding of the molecular mechanisms underlying oncogenic transformation. Thus, the ability to identify modification sites, *e.g.* phosphorylation sites, on a wide variety of cellular proteins is crucially important to understanding the key signaling proteins and pathways implicated in disease progression, as well as critical biological processes such as the immune response.

The efficient identification of protein phosphorylation sites relevant to signal transduction has been aided by the recent development of a powerful new class of antibodies, called motif-specific, context-independent antibodies, which are capable of specifically binding short, recurring signaling motifs comprising one or more modified (*e.g.* phosphorylated) amino acids in many different proteins in which the motif recurs. See U.S. Patent No. 6,441,140, Comb et al*.* Many of these powerful new antibodies are now available commercially. See CELL SIGNALING TECHNOLOGY, INC. 2003-04 Catalogue. More recently, a powerful new method for employing such motif-specific antibodies in immunoaffinity techniques coupled with mass spectrometric analysis to rapidly identify modified peptides from complex biological mixtures has been described. See U.S. Patent Publication No. 20030044848, Rush et al*.*). Such techniques will enable the rapid elucidation of protein activation and phosphorylation events underlying diseases, like cancer, that are driven by disruptions in signal transduction, as well as those underlying critical biological processes such as the immune response.

The transmission of intracellular signaling resulting from binding of the T-lymphocyte receptor (T-cell receptor) to foreign antigen presented with the major histocompatability complex (MHC) on antigen presenting cells (APCs) is a process critical to the generation of a proper immune response in mammals. Antigen-specific T-cell binding via the T-cell receptor results in a kinase-mediated signaling cascade leading to cell-specific proliferation of the activated T-cells, and their participation in the immune response against foreign antigens and cells. Defects in T-cell signaling have been associated with T-cell acute lymphocytic leukemias. See Blume-Jensen et al., Nature 411: 355-365 (2001) (describing T cell receptor beta gene translocation next to the gene encoding the Lck tyrosine kinase gene, resulting in presumably constitutive activation of Lck).

T-cell receptor-induced signaling is mediated through a variety of second messengers, protein kinases and phosphatases, and other enzymes and intermediates. It is now known that binding of the human T-cell receptor to specific antigen-MHC complex results in the activation and/or recruitment of the Src-family kinases, Lck and Fyn, which in turn phosphorylate two critical tyrosine residues within the immunoreceptor tyrosine-based activation motifs (ITAMs) in the TCR-ξ invariant chain of the TCR complex. *See, e.g.* Mustelin et al., Biochem J. 371: 15-27 (2003); Pitcher et al., Trends in Immunol., 24: 554-560 (2003). This process may also involve the exclusion of protein tyrosine phosphatases that would down-regulate Lck and Fyn, as well as the exclusion of Csk kinase, which negatively regulates Lck and Fyn by phosphorylation at a conserved C-terminal tyrosine (Tyr505 in Lck and Try528 in Fyn). See Mustelin *et al., supra.*

Phosphorylation of the ITAMs renders them high-affinity ligands for the ZAP-70 kinase, wh ich is selectively recruited to the activated receptor complex, and (along with the kinase Syk) is subsequently activated by phosphorylation at tyrosine 493 (Tyr493) by Lck kinase. See Mustelin *et al.,* Pitcher *et al., supra.* Following its activation, ZAP-70, along with Syk, in turn phosphorylates other key downstream adaptor proteins (such as LAT) and effector proteins (such as SLP-76). Further, certain phosphorylated tyrosine sites in activated ZAP-70 provide key docking sites for SH-2 domain-containing effector proteins like Lck and Cbl, which participate in a complex cascade - involving Ca²⁺/InsP₃, Ras/Raf/ERK and RhoA pathways, ultimately leading to gene regulation and cell proliferation. See Mustelin *et al.,* Pitcher *et al., supra.*

Lucas et al, J Immunol 154(12): 6275-6284 (1995) as well as Nagasawa et al, J Immunol 158(11): 5146-5154 (1997) disclose that the protein kinase Cdk6, whose sequence was published by Meyerson et al, EMBO J 11 (8): 2909-2917 (1992) for the first time, is involved in T-cell activation as such, but none of them mentions tyrosine phosphorylation. lavarone et al, Nature 387: 417-422 (1997) disclose that TGF-beta inhibits Cdk6 via phosphorylation of tyrosine 24, but neither suggests a corresponding site-specific anti-phosphotyrosine antibody nor establishes any link to a T-cell receptor signalling pathway.

Although some of the signaling proteins and phosphorylation sites involved in proper T-cell receptor signaling have been identified, a clear picture of the precise proteins and phosphorylation sites involved in propagating this essential biological signal remains to be developed. For example, SHP1 phosphatase and Fyn kinase may be involved in the signaling cascade, but their precise role and substrates are unknown. See Mustelin *et al., supra.* Other Src-family protein tyrosine kinases, including the Tec-related kinases, Itk/Emt and Txk/Rlk, appear to be involved as well, but their precise role and substrates remains to be determined. Accordingly, the small number of T-cell receptor signaling pathway-related phosphorylation sites that have been identified to date do not facilitate a complete and accurate understanding of how this important biological signal is propagated. Indeed, it has recently been concluded that a major remaining challenge in T-cell biology is more precisely define the contribution of particular signaling molecules involved in the T-cell signaling, and to better understand the interplay between signaling molecules and pathways involved. *See* Mustelin *et al., supra.*

Accordingly, there is a continuing need to unravel the molecular mechanisms of T-cell receptor signaling by identifying the downstream signaling proteins mediating the cascade leading to proliferation of activated T-cells and their participation in the immune response. Identifying particular phosphorylation sites on such signaling proteins and providing new reagents, such as phospho-specific antibodies and AQUA peptides, to detect and quantify them remains particularly important to advancing our understanding of the biology of the critical T-cell signaling process. In turn, such advances would lead to a better understanding of diseases, such as T-cell acute lymphocytic leukemias, involving aberrant T cell signaling. See Blume-Jensen *et al.,* supra.

### SUMMARY OF THE INVENTION

The specification discloses 95 novel phosphorylation sites identified in signal transduction proteins and pathways involved in T-cell receptor signaling, and provides new reagents, including phosphorylation-site specific antibodies and AQUA peptides, for the selective detection and quantification of these phosphorylated sites/proteins. Also provided are methods of using the reagents for the detection and quantification of the disclosed phosphorylation sites.

Specifically, an aspect of the invention provides a method for detecting or quantifying a signaling protein that is tyrosine-phopsphorylated in T-cell receptor signaling pathways, the method comprising the step of utilizing one or more of the following reagents to detect or quantify one or more T-cell receptor signaling protein(s) corresponding to one or more of Rows 61 and 62 of Column A of Table 1 only when phosphorylated at the tyrosine listed in corresponding Column F of Table 1: an isolated phosphorylation site-specific antibody that specifically binds the protein only when phosphorylated at the tyrosine listed in corresponding Column F of Table 1, comprised within the phosphorylation site sequence SEQ ID NO: 60 or SEQ ID NO: 61 listed in corresponding Column G of Table 1, wherein the antibody does not bind said protein when not phosphorylated at said tyrosine; and/or a heavy-isotope labeled peptide (AQUA peptide) for the quantification of the protein, the labeled peptide comprising the phosphorylation site sequence SEQ ID NO: 60 or SEQ ID NO: 61 listed in corresponding Column G of Table 1, comprising the phosphorylated tyrosine listed in corresponding Column F of Table 1.

Another aspect of the invention provides an isolated phosphorylation site-specific antibody that specifically binds a human T-cell receptor signaling protein corresponding to Row 61 or Row 62 of Column A of Table 1 only when phosphorylated at the tyrosine listed in corresponding Column F of Table 1, comprised within the phosphorylatable peptide sequence listed in corresponding Column G of Table 1 (SEQ ID NOs: 60 and 61), wherein the antibody does not bind said signaling protein when not phosphorylated at said tyrosine.

A further aspect of the invention provides an immortalized cell line producing an antibody that specifically binds a human T-cell receptor signaling protein corresponding to Row 61 or Row 62 of Column A of Table 1 only when phosphorylated at the tyrosine listed in corresponding Column F of Table 1, comprised within the phosphorylatable peptide sequence listed in corresponding Column G of Table 1 (SEQ ID NOs: 60 and 61), wherein the antibody does not bind said signaling protein when not phosphorylated at said tyrosine. Optionally, the immortalized cell line is a rabbit hybridoma or a mouse hybridoma.

Another aspect of the invention provides a heavy-isotope labeled peptide (AQUA peptide) for the quantification of a human T-cell receptor signaling protein corresponding to one or more of Rows 61 and 62 of Column A of Table 1, the labeled peptide comprising the phosphorylatable peptide sequence SEQ ID NO: 60 or SEQ ID NO: 61 listed in corresponding Column G of Table 1, which sequence comprises the phosphorylatable tyrosine listed in corresponding Column F of Table 1.

### BRIEF DESCRIPTION OF THE DRAWINGS

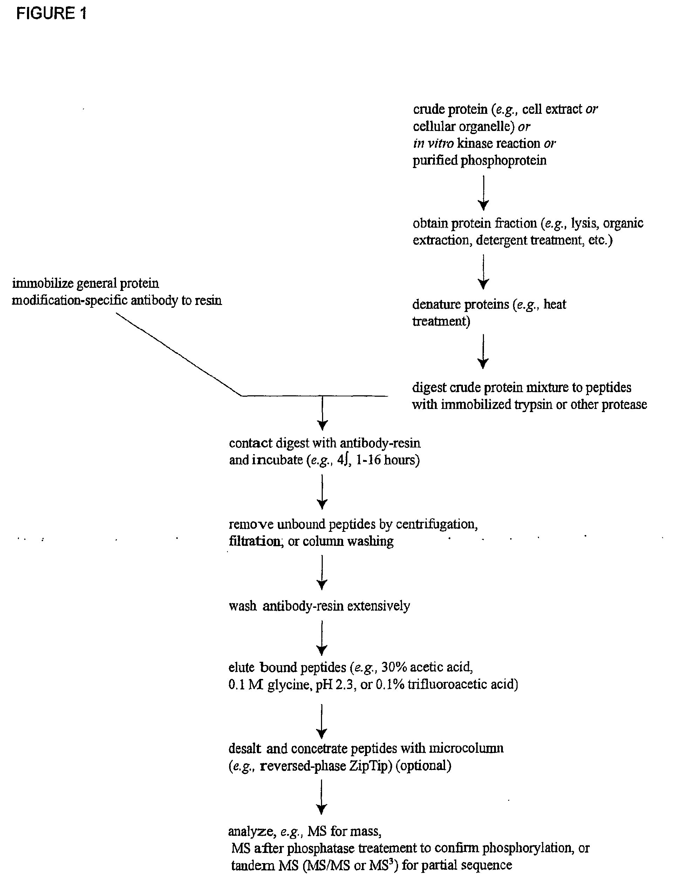

**FIG. 1** **-** Is a diagram broadly depicti ng the immunoaffinity isolation and mass-spectrometric characterization methodology (IAP) employed to identify the novel phosphorylation sites disclosed herein.

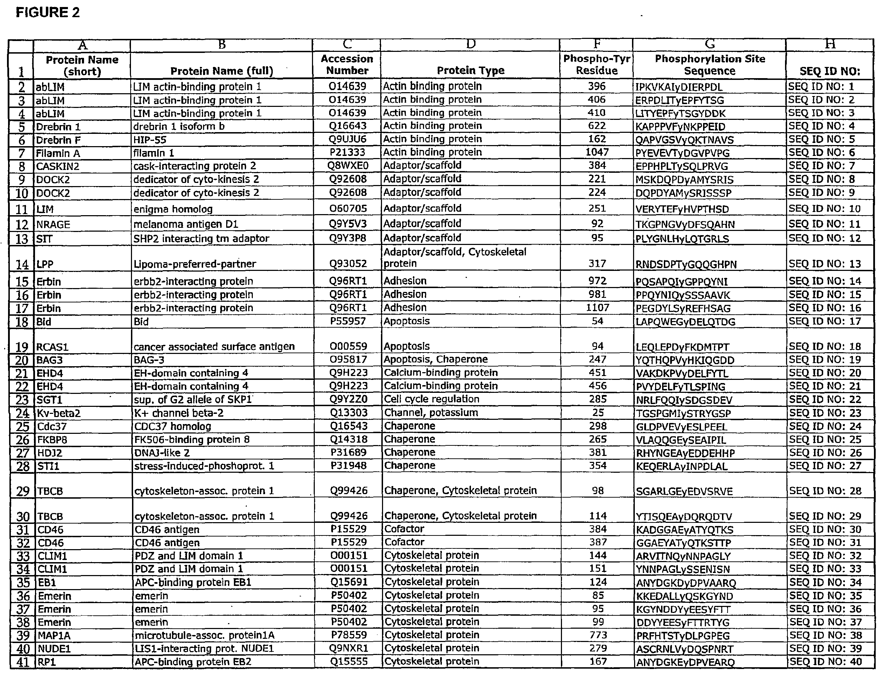

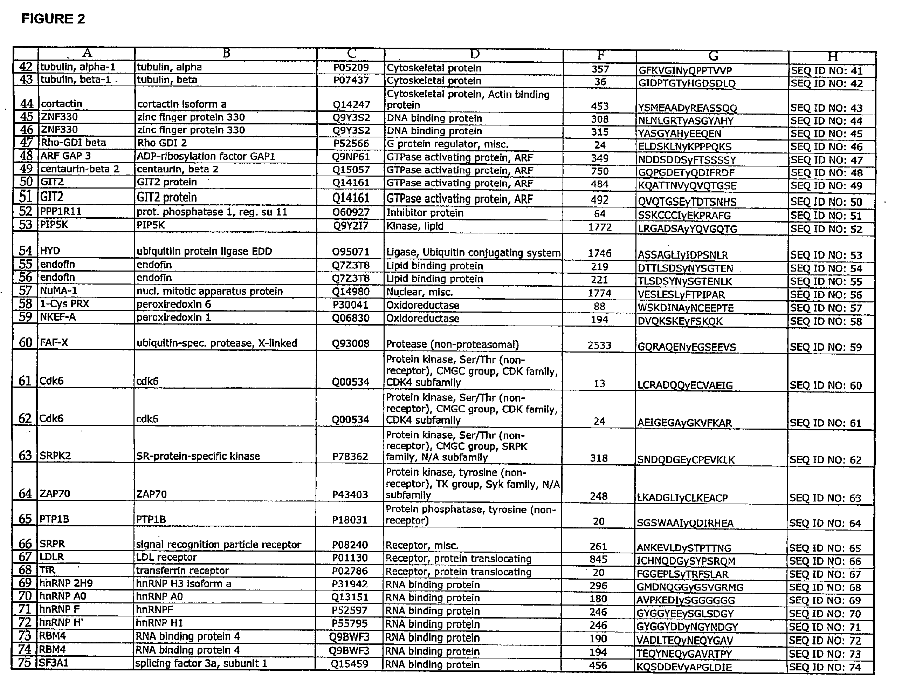

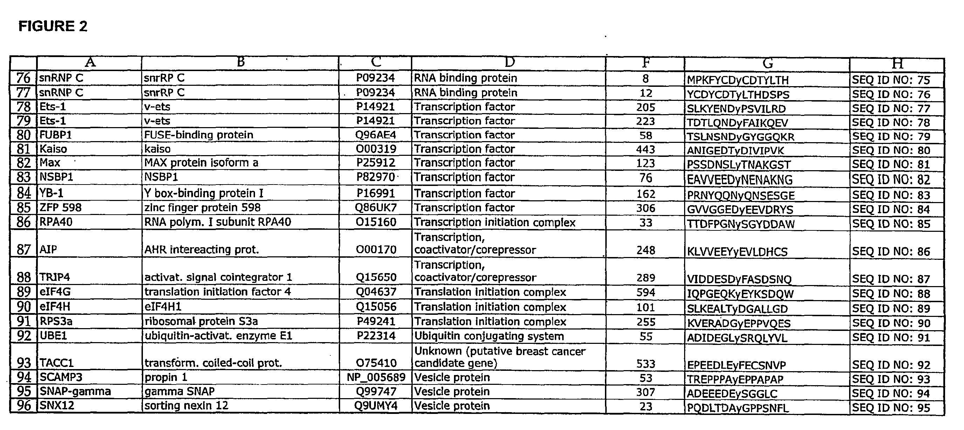

**FIG. 2** **-** Is a table (corresponding to Table 1) enumerating the T-cell receptor signaling protein phosphorylation sites disclosed herein: Column A = the abbreviated name of the parent protein; Column B = the full name of the parent protein; Column C = the SwissProt accession number for the protein (human sequence); Column D = the protein type/lassification; Column F = the residue (in the parent protein amino acid sequence) at which phosphorylation occurs within the phosphorylation site; and Column G = the phosphorylation site sequence encompassing the phosphorylatable residue; (tyrosine residue at which phosphorylation occurs (and corresponding to the respective entry in Column F) is indicated by lowercase "y".

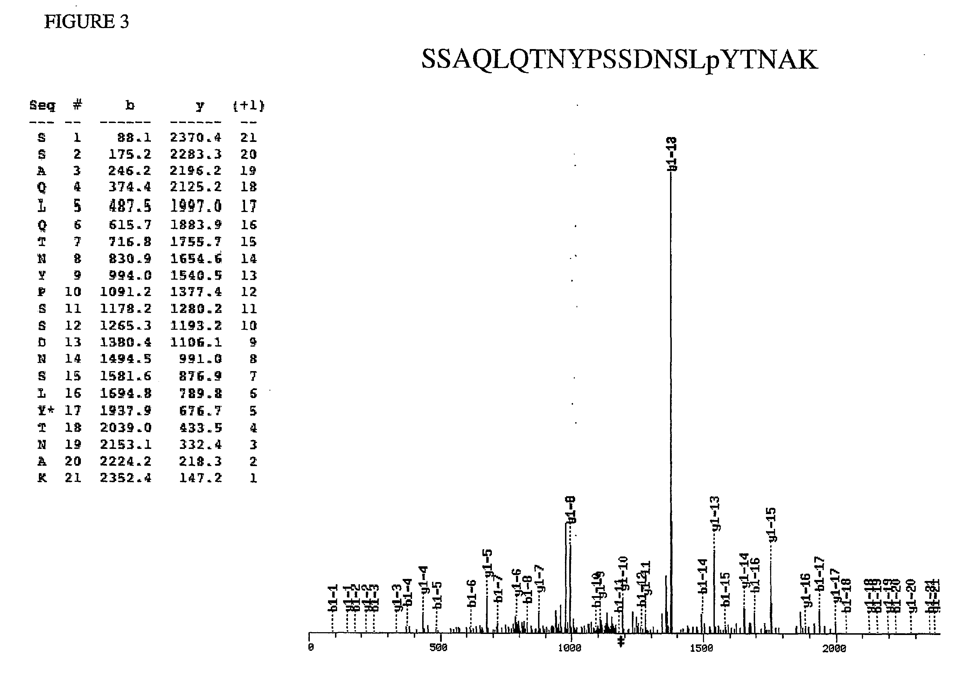

**FIG. 3** **-** is an exemplary mass spectrograph depicting the detection of the tyrosine 123 phosphorylation site in Max (see Row 82 in Figure 2/Table 1), as further described in Example 1 (red and blue indicate ions detected in MS/MS spectrum). The asterisk indicates the novel phosphotyrosine residue identified.

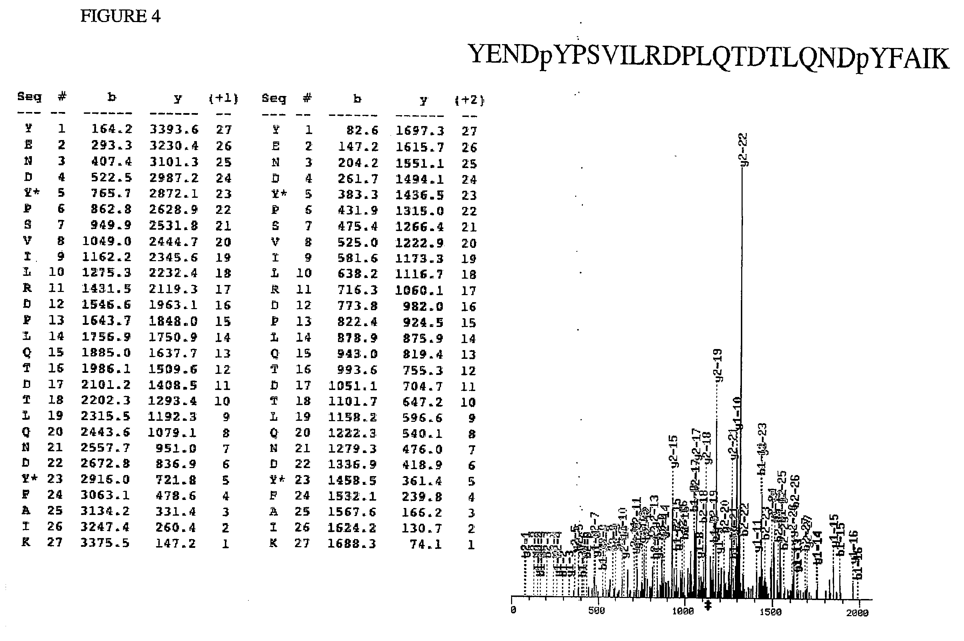

**FIG. 4** **-** is an exemplary mass spectrograph depicting the detection of the tyrosine 205 phosphorylation site in ETS-1 (see Row 78 in Figure 2/Table 1), as further described in Example 1 (red and blue indicate ions detected in MS/MS spectrum). The asterisk indicates the novel phosphotyrosine residue identified.

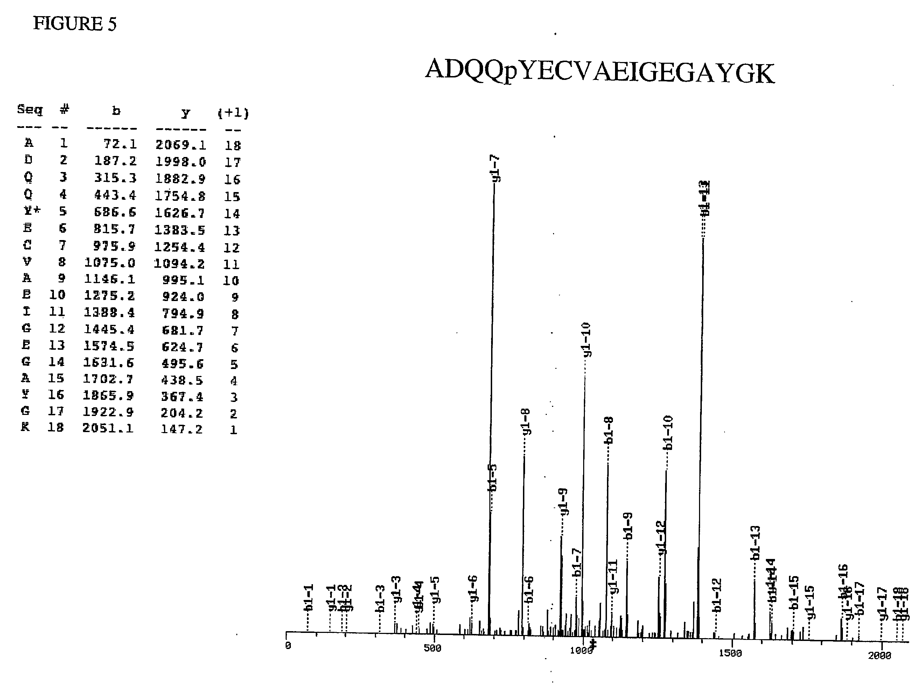

**FIG. 5** **-** is an exemplary mass spectrograph depicting the detection of the tyrosine 13 phosphorylation site in CDK6 (see Row 61 in Figure 2/Table 1), as further described in Example 1 (red and blue indicate ions detected in MS/MS spectrum). The asterisk indicates the novel phosphotyrosine residue identified.

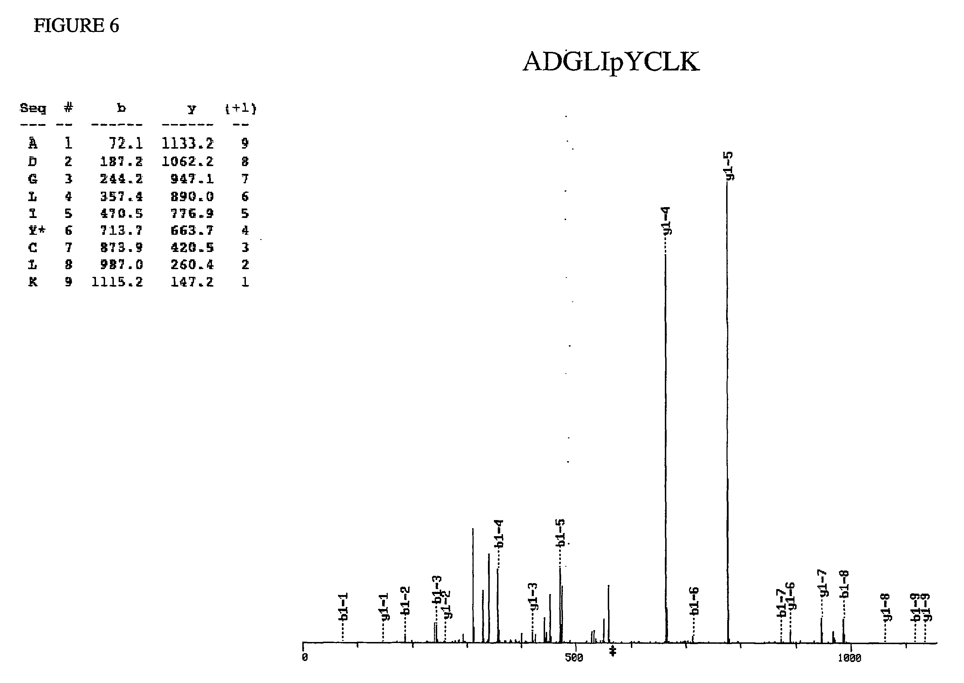

**FIG. 6** **-** is an exemplary mass spectrograph depicting the detection of the tyrosine 248 phosphorylation site in ZAP70 (see Row 64 in Figure 2/Table 1), as further described in Example 1 (red and blue indicate ions detected in MS/MS spectrum). The asterisk indicates the novel phosphotyrosine residue identified.

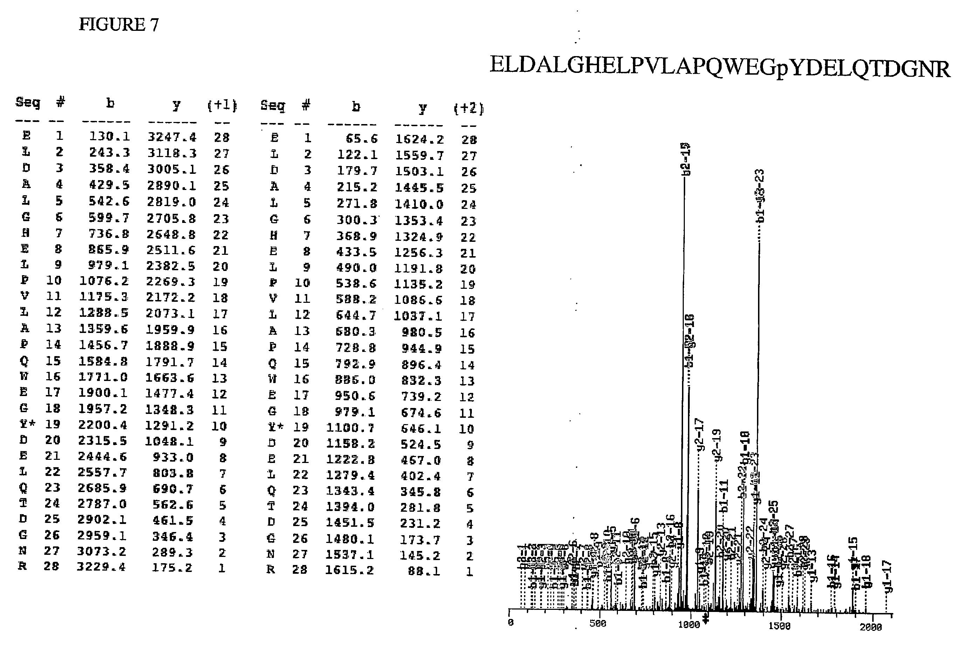

**FIG. 7** **-** is an exemplary mass spectrograph depicting the detection of the tyrosine 54 phosphorylation site in Bid (see Row 18 in Figure 2/ Table 1), as further described in Example 1 (red and blue indicate ions detected in MS/MS spectrum). The asterisk indicates the novel phosphotyrosine residue identified.

### DETAILED DESCRIPTION OF THE INVENTION

95 novel protein phosphorylation sites in signaling proteins and pathways involved in T-cell receptor signaling have now been discovered. These newly described phosphorylation sites were identified by employing the techniques described in "Immunoaffinity Isolation of Modified Peptides From Complex Mixtures," U.S. Patent Publication No. 20030044848, Rush et al.*,* using cellular extracts from an established cell line, derived from human lymphoblastic leukemia and non-Hodgkin lymphoma, in which T-cell signaling is activated, as further described below. The novel phosphorylation sites, and their corresponding parent proteins, disclosed herein are listed in Table I. These phosphorylation sites correspond to numerous different parent proteins (the full sequences of which (human) are all publicly available in SwissProt database and their Accession numbers listed in Column C of Table 1/Fig. 2), each of which fall into discrete protein type groups, for example Adaptor/Scaffold proteins, Chaperone proteins, Protein Kinases, and RNA Binding proteins, etc. (see Column D of Table 1), the phosphorylation of which is relevant to T-cell receptor signal transduction activity, as disclosed herein.

The discovery of the 95 novel protein phosphorylation sites described herein enables the production, by standard methods, of new reagents, such as phosphorylation site-specific antibodies and AQUA peptides (heavy-isotope labeled peptides), capable of specifically detecting and/or quantifying these phosphorylated sites/proteins. Such reagents are highly useful, *inter alia,* for studying signal tra nsduction events underlying the progression of diseases, such as acute lymphocytic leukemias, that may involve aberrant T-cell receptor signaling. Accordingly, the invention provides novel reagents -- phospho-specific antibodies and AQUA peptides -- for the specific detection and/or quantification of a T-cell receptor signaling protein/polypeptide only when phosphorylated (or only when not phosphorylated) at a particular phosphorylation site disclosed herein. Also provided are methods of detecting and/or quantifying one or more phosphorylated T-cell receptor signaling proteins using the phosphorylation-site specific antibodies and AQUA peptides.

In part, the specification provides an isolated phosphorylation site-specific antibody that specifically binds a given T-cell receptor signaling protein only when phosphorylated (or not phosphorylated, respectively) at a particular tyrosine enumerated in Column F of Table 1/Figure 2 comprised within the phosphorylatable peptide site sequence enumerated in corresponding Column G. In further part, the specification provides a heavy-isotope labeled peptide (AQUA peptide) for the qua ntification of a given T-cell receptor signaling protein, the labeled peptide comprising a particular phosphorylatable peptide site/sequence enumerated in Column G of Table 1/Figure 2 herein. For example, among the reagents provided by the invention is an isolated phosphorylation site-specific antibody that specifically binds the Cdk6 kinase (serine/threonine) only when phosphorylated (or only when not phosphorylated) at tyrosine 13 (see Row 61 (and Columns F and G) of Table 1/Figure 2). By way of further example, among the group of reagents provided by the invention is an AQUA peptide for the quantification of phosphorylated Cdk6 kinase, the AQUA peptide comprising the phosphorylatable peptide sequence listed in Column G, Row 61, of Table 1/Figure 2.

In one embodiment, the invention provides an isolated phosphorylation site-specific antibody that specifically binds a human T-cell receptor signaling protein corresponding to one or more of Rows 61 and 62 of Column A of Table 1 only when phosphorylated at the tyrosine listed in corresponding Column F of Table 1, comprised within the peptide sequence SEQ ID NO:60 or SEQ ID NO: 61 listed in corresponding Column G of Table 1, wherein said antibody does not bind said signaling protein when not phosphorylated at said tyrosine. In another embodiment, the invention provides an isolated phosphorylation site-specific antibody that specifically binds a T-cell receptor signaling protein corresponding to one or more of Rows 61 and 62 of Column A of Table 1 only when not phosphorylated at the tyrosine listed in corresponding Column F of Table 1, comprised within the peptide sequence SEQ ID NO: 60 or SEQ ID NO: 61 listed In corresponding Column G of Table 1 , wherein said antibody does not bind said signaling protein when phosphorylated at said tyrosine. Such reagents enable the specific detection of phosphorylation (or non-phosphorylation) of a novel phosphorylatable site disclosed herein. The invention further provides immortalized cell lines producing such antibodies. In one preferred embodiment, the immortalized cell line is a rabbit or mouse hybridoma.

In another embodiment, the invention provides a heavy-isotope labeled peptide (AQUA peptide) for the quantification of a T-cell receptor signaling protein corresponding to one or more of Rows 61 and 62 of Column A of Table 1, said labeled peptide comprising the phosphorylatable peptide sequence SEQ ID NO: 60 or SEQ ID NO: 61 listed in corresponding Column G of Table 1, which sequence comprises the phosphorylatable tyrosine listed in corresponding Column F of Table 1. In certain preferred embodiments, the phosphorylatable tyrosine within the labeled peptide is phosphorylated, while in other preferred embodiments, the phosphorylatable tyrosine within the labeled peptide is not phosphorylated.

Reagents (antibodies and AQUA peptides) may conveniently be grouped by the type of T-cell receptor signaling protein in which a given phosphorylation site (for which reagents are provided) occurs. The protein types for each respective protein (in which a phosphorylation site has been discovered) are provided in Column D of Table 1/Figure 2, and include: Actin Binding proteins, Adaptor/Scaffold proteins, Adhesion proteins, Calcium-binding proteins, Cell Cycle Regulation or Channel proteins, Chaperones, Cofactor proteins, Cytoskeletal proteins, DNA Binding proteins, G protein or GTPase Activating proteins, Ligases, Lipid Kinases and Binding proteins, Oxidoreductases, Protein Kinases, Protein Phosphatases, Receptor proteins, RNA Binding proteins, Transcription Factor/initiation Complex/Coactivator proteins, Translation Initiation Complex proteins, Ubitquitin Conjugating System proteins, and Vesicle proteins.

The invention also provides, in part, an immortalized cell line producing an antibody of the Invention . In one preferred embodiment, the immortalized cell line is a rabbit hybridoma or a mouse hybridoma.

In certain other preferred embodiments, a heavy-isotope labeled peptide (AQUA peptide) of the invention comprises a disclosed site sequence wherein the phosphorylatable tyrosine is phosphorylated. In certain other preferred embodiments, a heavy-isotope labeled peptide of the invention comprises a disclosed site sequence wherein the phosphorylatable tyrosine is *not* phosphorylated.

Also provided by the invention are methods for detecting or quantifying a T-cell receptor signaling protein that is tyrosine-phosphorylated, said method comprising the step of utilizing one or more of the above-described reagents of the invention to detect or quantify one or more T-cell receptor signaling protein(s) corresponding to one or more of Rows 61 and 62 of Column A of Table 1 only when phosphorylated at the tyrosine listed in corresponding Column F of Table 1.

The identification of the disclosed novel T-cell receptor signaling protein phosphorylation sites, and the standard production and use of the reagents provided by the invention are described in further detail below.

**Table 1. Newly-Discovered T-cell Receptor Signaling Protein Phosphorylation Sites.**

| | **A** | **C** | **D** | **F** | **G** | **H** |
|---|---|---|---|---|---|---|
| 1 | **Protein Name (short)** | **Accession Number** | **Protein Type** | **Phospho-Residue** | **Phosphorylation Site Sequence** | **SEQ ID NO:** |
| 2 | abLIM | 014639 | **Actin binding** protein | 396 | IPKVKAIyDIERPDL | SEQ ID NO: 1 |
| 3 | abLIM | 014639 | Actin binding protein | 406 | ERPDLITyEPFYTSG | SEQ ID NO: 2 |
| 4 | abLIM | 014639 | Actin binding protein | 410 | LITYEPFyTSGYDDK | SEQ ID NO: 3 |
| 5 | Drebrin 1 | Q16643 | Actin binding protein | 622 | KAPPPVFyNKPPEID | SEQ ID NO: 4 |
| 6 | Drebrin F | Q9UJU6 | Actin binding protein | 162 | QAPVGSVyQKTNAVS | SEQ ID NO: 5 |
| 7 | Filamin A | P21333 | Actin binding protein | 1047 | PYEVEVTyDGVPVPG | SEQ ID NO: 6 |
| 8 | CASKIN2 | Q8WXE0 | Adaptor/scaffold | 384 | EPPHPLTySQLPRVG | SEQ ID NO: 7 |
| 9 | DOCK2 | Q92608 | Adaptor/scaffold | 221 | MSKDQPDyAMYSRIS | SEQ ID NO: 8 |
| 10 | DOCK2 | Q92608 | Adaptor/scaffold | 224 | DQPDYAMySRISSSP | SEQ ID NO: 9 |
| 11 | LIM | 060705 | Adaptor/scaffold | 251 | VERYTEFyHVPTHSD | SEQ ID NO: 10 |
| 12 | NRAGE | Q9Y5V3 | Adaptor/scaffold | 92 | TKGPNGVyDFSQAHN | SEQ ID NO: 11 |
| 13 | SIT | Q9Y3P8 | Adaptor/scaffold | 95 | PLYGNLHyLQTGRLS | SEQ ID NO: 12 |
| 14 | LPP | Q93052 | Adaptor/scaffold, Cytoskeletal protein | 317 | RNDSDPTyGQQGHPN | SEQ ID NO: 13 |
| 15 | Erbin | Q96RT1 | Adhesion | 972 | PQSAPQIyGPPQYNI | SEQ ID NO: 14 |
| 16 | Erbin | Q96RT1 | Adhesion | 981 | PPQYNIQySSSAAVK | SEQ ID NO: 15 |
| 17 | Erbin | Q96RT1 | Adhesion | 1107 | PEGDYLSyREFHSAG | SEQ ID NO: 16 |
| 18 | Bid | P55957 | Apoptosis | 54 | LAPQWEGyDELQTDG | SEQ ID NO: 17 |
| 19 | RCAS1 | 000559 | Apoptosis | 94 | LEQLEPDyFKDMTPT | SEQ ID NO: 18 |
| 20 | BAG3 | 095817 | Apoptosis, Chaperone | 247 | YQTHQPVyHKIQGDD | SEQ ID NO: 19 |
| 21 | EHD4 | Q9H223 | Calcium-binding protein | 451 | VAKDKPVyDELFYTL | SEQ ID NO: 20 |
| 22 | EHD4 | Q9H223 | Calcium-binding protein | 456 | PVYDELFyTLSPING | SEQ ID NO: 21 |
| 23 | SGT1 | Q9Y2Z0 | Cell cycle regulation | 285 | NRLFQQIySDGSDEV | SEQ ID NO: 22 |
| 24 | Kv-beta2 | Q13303 | Channel, potassium | 25 | TGSPGMIySTRYGSP | SEQ ID NO: 23 |
| 25 | Cdc37 | Q16543 | Chaperone | 298 | GLDPVEVyESLPEEL | SEQ ID NO: 24 |
| 26 | FKBP8 | Q14318 | Chaperone | 265 | VLAQQGEySEAIPIL | SEQ ID NO: 25 |
| 27 | HDJ2 | P31689 | Chaperone | 381 | RHYNGEAyEDDEHHP | SEQ ID NO: 26 |
| 28 | STI1 | P31948 | Chaperone | 354 | KEQERLAyINPDLAL | SEQ ID NO: 27 |
| 29 | TBCB | Q99426 | Chaperone, Cytoskeletal protein | 98 | SGARLGEyEDVSRVE | SEQ ID NO: 28 |
| 30 | TBCB | Q99426 | Chaperone, Cytoskeletal protein | 114 | YTISQEAyDQRQDTV | SEQ ID NO: 29 |
| 31 | CD46 | P15529 | Cofactor | 384 | KADGGAEyATYQTKS | SEQ ID NO: 30 |
| 32 | CD46 | P15529 | Cofactor | 387 | GGAEYATyQTKSTTP | SEQ ID NO: 31 |
| 33 | CLIM1 | 000151 | Cytoskeletal protein | 144 | ARVITNQyNNPAGLY | SEQ ID NO: 32 |
| 34 | CLIM1 | 000151 | Cytoskeletal protein | 151 | YNNPAGLySSENISN | SEQ ID NO: 33 |
| 35 | EB1 | Q15691 | Cytoskeletal protein | 124 | ANYDGKDyDPVAARQ | SEQ ID NO: 34 |
| 36 | Emerin | P50402 | Cytoskeletal protein | 85 | KKEDALLyQSKGYND | SEQ ID NO: 35 |
| 37 | Emerin | P50402 | Cytoskeletal protein | 95 | KGYNDDYyEESYFTT | SEQ ID NO: 36 |
| 38 | Emerin | P50402 | Cytoskeletal protein | 99 | DDYYEESyFTTRTYG | SEQ ID NO: 37 |
| 39 | MAP1A | P78559 | Cytoskeletal protein | 773 | PRFHTSTyDLPGPEG | SEQ ID NO: 38 |
| 40 | NUDE1 | Q9NXR1 | Cytoskeletal protein | 279 | ASCRNLVyDQSPNRT | SEQ ID NO: 39 |
| 41 | RP1 | Q15555 | Cytoskeletal protein | 167 | ANYDGKEyDPVEARQ | SEQ ID NO: 40 |
| 42 | tubulin, alpha-1 | P05209 | Cytoskeletal protein | 357 | GFKVGINyQPPTVVP | SEQ ID NO: 41 |
| 43 | tubulin, beta-1 | P07437 | Cytoskeletal protein | 36 | GIDPTGTyHGDSDLQ | SEQ ID NO: 42 |
| 44 | cortactin | Q14247 | Cytoskeletal protein, Actin binding protein | 453 | YSMEAADyREASSQQ | SEQ ID NO: 43 |
| 45 | ZNF330 | Q9Y3S2 | DNA binding protein | 308 | NLNLGRTyASGYAHY | SEQ ID NO: 44 |
| 46 | ZNF330 | Q9Y3S2 | DNA binding protein | 315 | YASGYAHyEEQEN | SEQ ID NO: 45 |
| 47 | Rho-GDI beta | P52566 | G protein regulator, misc. | 24 | ELDSKLNyKPPPQKS | SEQ ID NO: 46 |
| 48 | ARF GAP 3 | Q9NP61 | GTPase activating protein, ARF | 349 | NDDSDDSyFTSSSSY | SEQ ID NO: 47 |
| 49 | centaurin-beta 2 | Q15057 | GTPase activating protein, ARF | 750 | GQPGDETyQDIFRDF | SEQ ID NO: 48 |
| 50 | GIT2 | Q14161 | GTPase activating protein, ARF | 484 | KQATTNVyQVQTGSE | SEQ ID NO: 49 |
| 51 | GIT2 | Q14161 | GTPase activating protein, ARF | 492 | QVQTGSEyTDTSNHS | SEQ ID NO: 50 |
| 52 | PPP1R11 | 060927 | Inhibitor protein | 64 | SSKCCCIyEKPRAFG | SEQ ID NO: 51 |
| 53 | PIP5K | Q9Y2I7 | Kinase, lipid | 1772 | LRGADSAyYQVGQTG | SEQ ID NO: 52 |
| 54 | HYD | 095071 | Ligase, Ubiquitin conjugating system | 1746 | ASSAGLIyIDPSNLR | SEQ ID NO: 53 |
| 55 | endofin | Q7Z3T8 | Lipid binding protein | 219 | DTTLSDSyNYSGTEN | SEQ ID NO: 54 |
| 56 | endofin | Q7Z3T8 | Lipid binding protein | 221 | TLSDSYNySGTENLK | SEQ ID NO: 55 |
| 57 | NuMA-1 | Q14980 | Nuclear, misc. | 1774 | VESLESLyFTPIPAR | SEQ ID NO: 56 |
| 58 | 1-Cys PRX | P30041 | Oxidoreductase | 88 | WSKDINAyNCEEPTE | SEQ ID NO: 57 |
| 59 | NKEF-A | Q06830 | Oxidoreductase | 194 | DVQKSKEyFSKQK | SEQ ID NO: 58 |
| 60 | FAF-X | Q93008 | Protease (non-proteasomal) | 2533 | GQRAQENyEGSEEVS | SEQ ID NO: 59 |
| 61 | Cdk6 | Q00534 | Protein kinase, Ser/Thr (non-receptor), CMGC group, CDK family, CDK4 subfamily | 13 | LCRADQQyECVAEIG | SEQ ID NO: 60 |
| 62 | Cdk6 | Q00534 | Protein kinase, Ser/Thr (non-receptor), CMGC group, CDK family, CDK4 subfamily | 24 | AEIGEGAyGKVFKAR | SEQ ID NO: 61 |
| 63 | SRPK2 | P78362 | Protein kinase, Ser/Thr (non-receptor), CMGC group, SRPK family, N/A subfamily | 318 | SNDQDGEyCPEVKLK | SEQ ID NO: 62 |
| 64 | ZAP70 | P43403 | Protein kinase, tyrosine (non-receptor), TK group, Syk family, N/A subfamily | 248 | LKADGLIyCLKEACP | SEQ ID NO: 63 |
| 65 | PTP1B | P18031 | Protein phosphatase, tyrosine (non-receptor) | 20 | SGSWAAIyQDIRHEA | SEQ ID NO: 64 |
| 66 | SRPR | P08240 | Receptor, misc. | 261 | ANKEVLDySTPTTNG | SEQ ID NO: 65 |
| 67 | LDLR | P01130 | Receptor, protein translocating | 845 | ICHNQDGySYPSRQM | SEQ ID NO: 66 |
| 68 | TfR | P02786 | Receptor, protein translocating | 20 | FGGEPLSyTRFSLAR | SEQ ID NO: 67 |
| 69 | hnRNP 2H9 | P31942 | RNA binding protein | 296 | GMDNQGGyGSVGRMG | SEQ ID NO: 68 |
| 70 | hnRNP A0 | Q13151 | RNA binding protein | 180 | AVPKEDIySGGGGGG | SEQ ID NO: 69 |
| 71 | hnRNP F | P52597 | RNA binding protein | 246 | GYGGYEEySGLSDGY | SEQ ID NO: 70 |
| 72 | hnRNP H' | P55795 | RNA binding protein | 246 | GYGGYDDyNGYNDGY | SEQ ID NO: 71 |
| 73 | RBM4 | Q9BWF3 | RNA binding protein | 190 | VADLTEQyNEQYGAV | SEQ ID NO: 72 |
| 74 | RBM4 | Q9BWF3 | RNA binding protein | 194 | TEQYNEQyGAVRTPY | SEQ ID NO: 73 |
| 75 | SF3A1 | Q15459 | RNA binding protein | 456 | KQSDDEVyAPGLDIE | SEQ ID NO: 74 |
| 76 | snRNP C | P09234 | RNA binding protein | 8 | MPKFYCDyCDTYLTH | SEQ ID NO: 75 |
| 77 | snRNP C | P09234 | RNA binding protein | 12 | YCDYCDTyLTHDSPS | SEQ ID NO: 76 |
| 78 | Ets-1 | P14921 | Transcription factor | 205 | SLKYENDyPSVILRD | SEQ ID NO: 77 |
| 79 | Ets-1 | P14921 | Transcription factor | 223 | TDTLQNDyFAIKQEV | SEQ ID NO: 78 |
| 80 | FUBP1 | Q96AE4 | Transcription factor | 58 | TSLNSNDyGYGGQKR | SEQ ID NO: 79 |
| 81 | Kaiso | 000319 | Transcription factor | 443 | ANIGEDTyDIVIPVK | SEQ ID NO: 80 |
| 82 | Max | P25912 | Transcription factor | 123 | PSSDNSLyTNAKGST | SEQ ID NO: 81 |
| 83 | NSBP1 | P82970 | Transcription factor | 76 | EAVVEEDyNENAKNG | SEQ ID NO: 82 |
| 84 | YB-1 | P16991 | Transcription factor | 162 | PRNYQQNyQNSESGE | SEQ ID NO: 83 |
| 85 | ZFP 598 | Q86UK7 | Transcription factor | 306 | GVVGGEDyEEVDRYS | SEQ ID NO: 84 |
| 86 | RPA40 | 015160 | Transcription initiation complex | 33 | TTDFPGNySGYDDAW | SEQ ID NO: 85 |
| 87 | AIP | O00170 | Transcription, coactivator/corep ressor | 248 | KLVVEEYyEVLDHCS | SEQ ID NO: 86 |
| 88 | TRIP4 | Q15650 | Transcription, coactivator/corep ressor | 289 | VIDDESDyFASDSNQ | SEQ ID NO: 87 |
| 89 | eIF4G | Q04637 | Translation initiation complex | 594 | IQPGEQKyEYKSDQW | SEQ ID NO: 88 |
| 90 | eIF4H | Q15056 | Translation initiation complex | 101 | SLKEALTyDGALLGD | SEQ ID NO: 89 |
| 91 | RPS3a | P49241 | Translation initiation complex | 255 | KVERADGyEPPVQES | SEQ ID NO: 90 |
| 92 | UBE1 | P22314 | Ubiquitin conjugating system | 55 | ADIDEGLySRQLYVL | SEQ ID NO: 91 |
| 93 | TACC1 | 075410 | Unknown (putative breast cancer candidate gene) | 533 | EPEEDLEyFECSNVP | SEQ ID NO: 92 |
| 94 | SCAMP3 | NP_00568 9 | Vesicle protein | 53 | TREPPPAyEPPAPAP | SEQ ID NO: 93 |
| 95 | SNAP-gamma | Q99747 | Vesicle protein | 307 | ADEEEDEySGGLC | SEQ ID NO: 94 |
| 96 | SNX12 | Q9UMY4 | Vesicle protein | 23 | PQDLTDAyGPPSNFL | SEQ ID NO: 95 |

The short name for each protein in which a phosphorylation site has presently been identified is provided in Column A, and it accession number (human) is provided Column C. The protein type/group into which each protein falls is provided in Column D. The identified tyrosine residue at which phosphorylation occurs in a given protein is identified in Column F, and the amino acid sequence of the phosphorylation site encompassing the tyrosine residue is provided in Column G (lower case y = the tyrosine (identified in Column F) at which phosphorylation occurs. Table 1 above is identical to Figure 2, except that the latter includes the full protein name (Column B).

The identification of these 95 phosphorylation sites is described in more detail in Part A below and in Example 1.

### Definitions.

As used herein, the following terms have the meanings indicated:
"Antibody" or "antibodies" refers to all types of immunoglobulins, including IgG, IgM, IgA, IgD, and IgE, including F_{ab} or antigen-recognition fragments thereof, including chimeric, polyclonal, and monoclonal antibodies. The term "does not bind" with respect to an antibody's binding to one phospho-form of a sequence means does not substantially react with as compared to the antibody's binding to the other phospho-form of the sequence for which the antibody is specific.
"T-cell receptor signaling protein" means any protein (or polypeptide derived therefrom) enumerated in Column A of Table 1/Figure 2, which is disclosed herein as being phosphorylated in one or more cell line(s) in which T-cell receptor signaling is activated. T-cell receptor signaling proteins may be direct substrates of T-cell receptor itself, or may be indirect substrates downstream in T-cell receptor signaling pathways. A T-cell receptor signaling protein may also be phosphorylated in other cell lines harboring activated kinase activity.
"Heavy-isotope labeled peptide" (used interchangeably with AQUA peptide) means a peptide comprising at least one heavy-isotope label, which is suitable for absolute quantification or detection of a protein as described in WO/03016861, "Absolute Quantification of Proteins and Modified Forms Thereof by Multistage Mass Spectrometry" (Gygi *et al.*), further discussed below.
"Protein" is used interchangeably with polypeptide, and includes protein fragments and domains as well as whole protein.
"Phosphorylatable amino acid" means any amino acid that is capable of being modified by addition of a phosphate group, and includes both forms of such amino acid.
"Phosphorylatable peptide sequence" means a peptide sequence comprising a phosphorylatable amino acid.
"Phosphorylation site-specific antibody" means an antibody that specifically binds a phosphorylatable peptide sequence/epitope only when phosphorylated, or only when not phosphorylated, respectively. The term is used interchangeably with "phospho-specific" antibody.

### A. Identification of Novel T-cell Receptor Signaling Protein Phosphorylation Sites.

The 95 novel T-cell receptor signaling protein phosphorylation sites disclosed herein and listed in Table 1/Figure 2 were discovered by employing the modified peptide isolation and characterization techniques described in described in "Immunoaffinity Isolation of Modified Peptides From Complex Mixtures," U.S. Patent Publication No. 20030044848, Rush et al*.* using cellular extracts from a Jurkat cell line in which the T-cell receptor signaling is constitutively activated. The isolation and identification of phosphopeptides from this T-cell line, using an immobilized general phosphotyrosine-specific antibody, is described in detail in Example 1 below. In addition to the 95 previously unknown protein phosphorylation sites discovered, many known phosphorylation sites were also identified (not described herein). The immunoaffinity/mass spectrometric technique described in the '848 Patent Publication (the "IAP" method) -- and employed as described in detail in the Examples -- is briefly summarized below.

The IAP method employed generally comprises the following steps: (a) a proteinaceous preparation (*e.g.* a digested cell extract) comprising phosphopeptides from two or more different proteins is obtained from an organism; (b) the preparation is contacted with at least one immobilized general phosphotyrosine-specific antibody; (c) at least one phosphopeptide specifically bound by the immobilized antibody in step (b) is isolated; and (d) the modified peptide isolated in step (c) is characterized by mass spectrometry (MS) and/or tandem mass spectrometry (MS-MS). Subsequently, (e) a search program (*e.g.* Sequest) may be utilized to substantially match the spectra obtained for the isolated, modified peptide during the characterization of step (d) with the spectra for a known peptide sequence. A quantification step employing, *e.g.* SILAC or AQUA, may also be employed to quantify isolated peptides in order to compare peptide levels in a sample to a baseline.

In the IAP method as employed herein, a general phosphotyrosine-specific monoclonal antibody (commercially available from Cell Signaling Technology, Inc., Beverly, MA, Cat #9411 (p-Tyr-100)) was used in the immunoaffinity step to isolate the widest possible number of phosphotyrosine containing peptides from the T-cell extracts.

Extracts from a pervanadate-treated Jurkat cell line were employed. This established cell line is derived from patients with acute lymphoblastic leukemia and leukemic transformed non-Hodgkin lymphoma, in which T-cell receptor signaling pathways are constitutively activated.

As described in more detail in the Examples, lysates were prepared from this cell line and digested with trypsin after treatment with DTT and iodoacetamide to alkylate cysteine residues. Before the immunoaffinity step, peptides were pre-fractionated by reversed-phase solid phase extraction using Sep-Pak C₁₈ columns to separate peptides from other cellular components. The solid phase extraction cartridges were eluted with varying steps of acetonitrile. Each lyophilized peptide fraction was redissolved in PBS and treated with phosphotyrosine antibody (P-Tyr-100, CST #9411) immobilized on protein G-Sepharose. Immunoaffinity-purified peptides were eluted with 0.1 % TFA and a portion of this fraction was concentrated with Stage tips and analyzed by LC-MS/MS, using a ThermoFinnigan LCQ Deca XP Plus ion trap mass spectrometer. Peptides were eluted from a 10 cm x 75 µm reversed-phase column with a 45-min linear gradient of acetonitrile. MS/MS spectra were evaluated using the program Sequest with the NCBI human protein database.

This revealed a total of 95 novel tyrosine phosphorylation sites in signaling pathways affected by T-cell receptor activation. The identified phosphorylation sites and their parent proteins are enumerated in Table 1/Figure 2. The tyrosine (human sequence) at which phosphorylation occurs is provided in Column F, and the peptide sequence encompassing the phosphorylatable tyrosine residue at the site is provided in Column G.

As a result of the discovery of these phosphorylation sites, phospho-specific antibodies and AQUA peptides for the detection of and quantification of these sites and their parent proteins may now be produced by standard methods, described below. These new reagents will prove highly useful in studying the signaling pathways and events underlying the progression of diseases mediated by altered T-cell receptor signaling and the identification of new biomarkers and targets for diagnosis and treatment of such diseases.

### B. Antibodies and Cell Lines

Isolated phosphorylation site-specific antibodies that specifically bind a T-cell receptor signaling protein disclosed in Column A of Table 1 only when phosphorylated (or only when not phosphorylated) at the corresponding amino acid (tyrosine) and phosphorylation site listed in Columns F and G of Table 1 may now be produced by standard antibody production methods, such as anti-peptide antibody methods, using the phosphorylation site sequence information provided in Column G of Table 1. For example, two previously unknown Cdk6 kinase phosphorylation sites (tyrosines 13 and 24) (see Rows 61-62 of Table 1) are presently disclosed. Thus, antibodies that specifically bind any one of these novel Cdk6 sites can now be produced by using (all or part of) the amino acid sequence encompassing the respective phosphorylated residue as a peptide antigen used to immunize an animal (*e.g.* a peptide antigen comprising the sequence set forth in Row 61, Column G, of Table 1 (which encompasses the phosphorylated tyrosine at position 13 in Cdk6) may be employed to produce an antibody that only binds Cdk6 when phosphorylated at Tyr13).

Polyclonal antibodies of the invention may be produced according to standard techniques by immunizing a suitable animal (*e.g.*, rabbit, goat, etc.) with a peptide antigen corresponding to the T-cell receptor protein phosphorylation site of interest (*i.e.* a phosphorylation site enumerated in Column G of Table 1, which comprises the corresponding phosphorylatable amino acid listed in Column F of Table 1), collecting immune serum from the animal, and separating the polyclonal antibodies from the immune serum, in accordance with known procedures. Similarly, a peptide comprising any of the phosphorylation site sequences provided in Column G of Table 1 may employed as an antigen to produce an antibody that only binds the corresponding protein listed in Column A of Table 1 when phosphorylated (or when *not* phosphorylated) at the corresponding residue listed in Column F. If an antibody that only binds the protein when phosphorylated at the disclosed site is desired, the peptide antigen includes the phosphorylated form of the amino acid. Conversely, if an antibody that only binds the protein when not phosphorylated at the disclosed site is desired, the peptide antigen includes the non-phosphorylated form of the amino acid.

Peptide antigens suitable for producing antibodies of the invention may be designed, constructed and employed in accordance with well-known techniques. See, *e.g.*, ANTIBODIES: A LABORATORY MANUAL, Chapter 5, p. 75-76, Harlow & Lane Eds., Cold Spring Harbor Laboratory (1988); Czernik, Methods In Enzymology, 201: 264-283 (1991); Merrifield, J. Am. Chem. Soc. 85: 21-49 (1962)).

It will be appreciated by those of skill in the art that longer or shorter phosphopeptide antigens may be employed. *See Id.* For example, a peptide antigen may consist of the full sequence disclosed in Column G of Table 1, or it may comprise additional amino acids flanking such disclosed sequence, or may comprise of only a portion of the disclosed sequence immediately flanking the phosphorylatable amino acid (indicated in Column G by lowercase "y"). Polyclonal antibodies produced as described herein may be screened as further described below.

Monoclonal antibodies of the invention may be produced in a hybridoma cell line according to the well-known technique of Kohler and Milstein. Nature 265: 495-97 (1975); Kohler and Milstein, Eur. J. Immunol. 6: 511 (1976); *see also,* CURRENT PROTOCOLS IN MOLECULAR BIOLOGY, Ausubel et al. Eds. (1989). Monoclonal antibodies so produced are highly specific, and improve the selectivity and specificity of diagnostic assay methods provided by the invention. For example, a solution containing the appropriate antigen may be injected into a mouse or other species and, after a sufficient time (in keeping with conventional techniques), the animal is sacrificed and spleen cells obtained. The spleen cells are then immortalized by fusing them with myeloma cells, typically in the presence of polyethylene glycol, to produce hybridoma cells. Rabbit fusion hybridomas, for example, may be produced as described in U.S Patent No. 5,675,063, C. Knight, Issued October 7, 1997. The hybridoma cells are then grown in a suitable selection media, such as hypoxanthine-aminopterin-thymidine (HAT), and the supernatant screened for monoclonal antibodies having the desired specificity, as described below. The secreted antibody may be recovered from tissue culture supernatant by conventional methods such as precipitation, ion exchange or affinity chromatography, or the like.

Monoclonal Fab fragments may also be produced in *Escherichia coli* by recombinant techniques known to those skilled in the art. See, *e.g.,* W. Huse, Science 246: 1275-81 (1989); Mullinax et al., Proc. Nat'l Acad. Sci. 87: 8095 (1990). If monoclonal antibodies of one isotype are preferred for a particular application, particular isotypes can be prepared directly, by selecting from the initial fusion, or prepared secondarily, from a parental hybridoma secreting a monoclonal antibody of different isotype by using the sib selection technique to isolate class-switch variants (Steplewski, et al., Proc. Nat'l. Acad. Sci., 82: 8653 (1985); Spira et al., J. Immuno/. Methods, 74: 307 (1984)).

The preferred epitope of a phosphorylation-site specific antibody of the invention is a peptide fragment consisting essentially of about 8 to 17 amino acids including the phosphorylatable tyrosine, wherein about 3 to 8 amino acids are positioned on each side of the phosphorylatable tyrosine , and antibodies of the invention thus specifically bind a target T-cell receptor signaling polypeptide comprising such epitopic sequence. Particularly preferred epitopes bound by the antibodies of the invention comprise all or part of a phosphorylatable site sequence listed in Column G of Table 1, including the phosphorylatable amino acid (tyrosine).

Included are equivalent non-antibody molecules, such as protein binding domains or nucleic acid aptamers, which bind, in a phospho-specific manner, to essentially the same phosphorylatable epitope to which the phospho-specific antibodies of the invention bind. *See, e.g.,* Neuberger et al., Nature 312: 604 (1984). Such equivalent non-antibody reagents may be suitably employed in the methods further described below.

Antibodies provided by the invention may be any type of immunoglobulins, including IgG, IgM, IgA, IgD, and IgE, including F_{ab} or antigen-recognition fragments thereof. The antibodies may be monoclonal or polyclonal and may be of any species of origin, including (for example) mouse, rat, rabbit, horse, or human, or may be chimeric antibodies. See, *e.g.,* M. Walker et al., Molec. Immuno/. 26: 403-11 (1989); Morrision et al., Proc. Nat'l. Acad. Sci. 81: 6851 (1984); Neuberger et al., Nature 312: 604 (1984)). The antibodies may be recombinant monoclonal antibodies produced according to the methods disclosed in U.S. Pat. No. 4,474,893 (Reading) or U.S. Pat. No. 4,816,567 (Cabilly et al.) The antibodies may also be chemically constructed by specific antibodies made according to the method disclosed in U.S. Pat. No. 4,676,980 (Segel et al.)

The invention also provides immortalized cell lines that produce an antibody of the invention. For example, hybridoma clones, constructed as described above, that produce monoclonal antibodies to the T-cell receptor signaling protein phosphorylation sties disclosed herein are also provided. Similarly, the invention includes recombinant cells producing an antibody of the invention, which cells may be constructed by well known techniques; for example the antigen combining site of the monoclonal antibody can be cloned by PCR and single-chain antibodies produced as phage-displayed recombinant antibodies or soluble antibodies in *E*. *coli* (*see, e.g.,* ANTIBODY ENGINEERING PROTOCOLS, 1995, Humana Press, Sudhir Paul editor.)

Phosphorylation site-specific antibodies of the invention, whether polyclonal or monoclonal, may be screened for epitope and phospho-specificity according to standard techniques. *See*, *e.g.* Czernik et al., Methods in Enzymology, 201: 264-283 (1991). For example, the antibodies may be screened against the phospho and non-phospho peptide library by ELISA to ensure specificity for both the desired antigen (*i.e*. that epitope including a phosphorylation site sequence enumerated in Column G of Table 1) and for reactivity only with the phosphorylated (or non-phosphorylated) form of the antigen. Peptide competition assays may be carried out to confirm lack of reactivity with other phosphoepitopes on the given T-cell receptor signaling protein. The antibodies may also be tested by Western blotting against cell preparations containing the signaling protein, *e*.*g*. cell lines over-expressing the target protein, to confirm reactivity with the desired phosphorylated epitope/target.

Specificity against the desired phosphorylated epitope may also be examined by constructing mutants lacking phosphorylatable residues at positions outside the desired epitope known to be phosphorylated, or by mutating the desired phospho-epitope and confirming lack of reactivity. Phosphorylation-site specific antibodies of the invention may exhibit some limited cross-reactivity related epitopes in non-target proteins. This is not unexpected as most antibodies exhibit some degree of cross-reactivity, and anti-peptide antibodies will often cross-react with epitopes having high homology to the immunizing peptide. *See*, *e.g*., *Czernik*, *supra*. Cross-reactivity with non-target proteins is readily characterized by Western blotting alongside markers of known molecular weight. Amino acid sequences of cross-reacting proteins may be examined to identify sites highly homologous to the T-cell receptor signaling protein epitope for which the antibody of the invention is specific. In certain cases, polyclonal antisera may be exhibit some undesirable general cross-reactivity to phosphotyrosine, which may be removed by further purification of antisera, *e*.*g*. over a phosphotyramine column. Antibodies of the invention specifically bind their target protein (*i*.*e*. a protein listed in Column A of Table 1/Figure 2) only when phosphorylated (or only when not phosphorylated, as the case may be) at the site disclosed in corresponding Columns F/G, and do not (substantially) bind to the other form (as compared to the form for which the antibody is specific).

Antibodies may be further characterized via immunohistochemical (IHC) staining using normal and diseased tissues to examine T-cell receptor phosphorylation and activation status in diseased tissue. IHC may be carried out according to well-known techniques. *See*, *e.g*., ANTIBODIES: A LABORATORY MANUAL, Chapter 10, Harlow & Lane Eds., Cold Spring Harbor Laboratory (1988). Briefly, paraffin-embedded tissue (*e.g*. tumor tissue) is prepared for immunohistochemical staining by deparaffinizing tissue sections with xylene followed by ethanol; hydrating in water then PBS; unmasking antigen by heating slide in sodium citrate buffer; incubating sections in hydrogen peroxide; blocking in blocking solution; incubating slide in primary antibody and secondary antibody; and finally detecting using ABC avidin/biotin method according to manufacturer's instructions.

Antibodies may be further characterized by flow cytometry carried out according to standard methods. *See* Chow et al., Cytometry (Communications in Clinical Cytometry) 46: 72-78 (2001). Briefly and by way of example, the following protocol for cytometric analysis may be employed: samples may be centrifuged on Ficoll gradients to remove erythrocytes, and cells may then be fixed with 2% paraformaldehyde for 10 minutes at 37°C followed by permeabilization in 90% methanol for 30 minutes on ice. Cells may then be stained with the primary phosphorylation-site specific antibody of the invention (which detects an T-cell receptor signal transduction protein enumerated in Table 1), washed and labeled with a fluorescent-labeled secondary antibody. Additional fluorochrome-conjugated marker antibodies (*e*.*g*. CD45, CD34) may also be added at this time to aid in the subsequent identification of specific hematopoietic cell types. The cells would then be analyzed on a flow cytometer (*e*.*g*. a Beckman Coulter FC500) according to the specific protocols of the instrument used.

Antibodies of the invention may also be advantageously conjugated to fluorescent dyes (*e*.*g*. Alexa488, PE) for use in multiparametric analyses along with other signal transduction (phospho-CrkL, phospho-Erk 1/2) and/or cell marker (CD34) antibodies.

Phosphorylation-site specific antibodies of the invention specifically bind to a human T-cell receptor signal transduction protein or polypeptide only when phosphorylated at a disclosed site, but are not limited only to binding the human species, *per se*. The invention includes antibodies that also bind conserved and highly-homologous or identical phosphorylation sites in respective T-cell receptor signaling proteins from other species (*e*.*g*. mouse, rat, monkey, yeast), in addition to binding the human phosphorylation site. Highly-homologous sites conserved in other species can readily be identified by standard sequence comparisons, such as using BLAST, with the human T-cell receptor signal transduction protein phosphorylation sites disclosed herein.

### C. Heavy-Isotope Labeled Peptides (AQUA Peptides).

The novel T-cell receptor signaling protein phosphorylation sites disclosed herein now enable the production of corresponding heavy-isotope labeled peptides for the absolute quantification of such signaling proteins (both phosphorylated and not phosphorylated at a disclosed site) in biological samples. The production and use of AQUA peptides for the absolute quantification of proteins (AQUA) in complex mixtures has been described. *See* WO/03016861, "Absolute Quantification of Proteins and Modified Forms Thereof by Multistage Mass Spectrometry," Gygi *et al*. and also Gerber et al. Proc. Natl. Acad. Sci. U.S.A. 100: 6940-5 (2003).

The AQUA methodology employs the introduction of a known quantity of at least one heavy-isotope labeled peptide standard (which has a unique signature detectable by LC-SRM chromatography) into a digested biological sample in order to determine, by comparison to the peptide standard, the absolute quantity of a peptide with the same sequence and protein modification in the biological sample. Briefly, the AQUA methodology has two stages: peptide Internal standard selection and validation and method development; and implementation using validated peptide internal standards to detect and quantify a target protein in sample. The method is a powerful technique for detecting and quantifying a given peptide/protein within a complex biological mixture, such as a cell lysate, and may be employed, *e*.*g*., to quantify change in protein phosphorylation as a result of drug treatment, or to quantify differences in the level of a protein in different biological states.

Generally, to develop a suitable internal standard, a particular peptide (or modified peptide) within a target protein sequence is chosen based on its amino acid sequence and the particular protease to be used to digest. The peptide is then generated by solid-phase peptide synthesis such that one residue is replaced with that same residue containing stable isotopes (¹³C, ¹⁵N). The result is a peptide that is chemically identical to its native counterpart formed by proteolysis, but is easily distinguishable by MS via a 7-Da mass shift. The newly synthesized AQUA internal standard peptide is then evaluated by LC-MS/MS. This process provides qualitative information about peptide retention by reverse-phase chromatography, ionization efficiency, and fragmentation via collision-induced dissociation. Informative and abundant fragment ions for sets of native and internal standard peptides are chosen and then specifically monitored in rapid succession as a function of chromatographic retention to form a selected reaction monitoring (LC-SRM) method based on the unique profile of the peptide standard.

The second stage of the AQUA strategy is its implementation to measure the amount of a protein or modified protein from complex mixtures. Whole cell lysates are typically fractionated by SDS-PAGE gel electrophoresis, and regions of the gel consistent with protein migration are excised. This process is followed by in-gel proteolysis in the presence of the AQUA peptides and LC-SRM analysis. (*See* Gerber *et al. supra*.) AQUA peptides are spiked in to the complex peptide mixture obtained by digestion of the whole cell lysate with a proteolytic enzyme and subjected to immunoaffinity purification as described above. The retention time and fragmentation pattern of the native peptide formed by digestion (e.g. trypsinization) is identical to that of the AQUA internal standard peptide determined previously; thus, LC-MS/MS analysis using an SRM experiment results in the highly specific and sensitive measurement of both internal standard and analyte directly from extremely complex peptide mixtures. Because an absolute amount of the AQUA peptide is added (*e*.*g*. 250 fmol), the ratio of the areas under the curve can be used to determine the precise expression levels of a protein or phosphorylated form of a protein in the original cell lysate. In addition, the internal standard is present du ring in-gel digestion as native peptides are formed, such that peptide extraction efficiency from gel pieces, absolute losses during sample handling (including vacuum centrifugation), and variability during introduction into the LC-MS system do not affect the determined ratio of native and AQUA peptide abundances.

An AQUA peptide standard is developed for a known phosphorylation site sequence previously identified by the IAP-LC-MS/MS method within in a target protein. One AQUA peptide incorporating the phosphorylated form of the particular residue within the site may be developed, and a second AQUA peptide incorporating the non-phosphorylated form of the residue developed. In this way, the two standards may be used to detect and quantify both the phosphorylated and non-phosphorylated forms of the site in a biological sample.

Peptide internal standards may also be generated by examining the primary amino acid sequence of a protein and determining the boundaries of peptides produced by protease cleavage. Alternatively, a protein may actually be digested with a protease and a particular peptide fragment produced can then sequenced. Suitable proteases include, but are not limited to, serine proteases (*e*.*g*. trypsin, hepsin), metallo proteases (*e*.*g*. PUMP1), chymotrypsin, cathepsin, pepsin, thermolysin, carboxypeptidases, etc.

A peptide sequence within a target protein is selected according to one or more criteria to optimize the use of the peptide as an internal standard. Preferably, the size of the peptide is selected to minimize the chances that the peptide sequence will be repeated elsewhere in other non-target proteins. Thus, a peptide is preferably at least about 6 amino acids. The size of the peptide is also optimized to maximize ionization frequency. Thus, peptides longer than about 20 amino acids are not preferred. The preferred ranged is about 7 to 15 amino acids. A peptide sequence is also selected that is not likely to be chemically reactive during mass spectrometry, thus sequences comprising cysteine, tryptophan, or methionine are avoided.

A peptide sequence that does not include a modified region of the target region may be selected so that the peptide internal standard can be used to determine the quantity of all forms of the protein. Alternatively, a peptide internal standard encompassing a modified amino acid may be desirable to detect and quantify only the modified form of the target protein. Peptide standards for both modified and unmodified regions can be used together, to determine the extent of a modification in a particular sample (*i*.*e*. to determine what fraction of the total amount of protein is represented by the modified form). For example, peptide standards for both the phosphorylated and unphosphorylated form of a protein known to be phosphorylated at a particular site can be used to quantify the amount of phosphorylated form in a sample.

The peptide is labeled using one or more labeled amino acids (*i*.*e*. the label is an actual part of the peptide) or less preferably, labels may be attached after synthesis according to standard methods. Preferably, the label is a mass-altering label selected based on the following considerations: The mass should be unique to shift fragments masses produced by MS analysis to regions of the spectrum with low background; the ion mass signature component is the portion of the labeling moiety that preferably exhibits a unique ion mass signature in MS analysis; the sum of the masses of the constituent atoms of the label is preferably uniquely different than the fragments of all the possible amino acids. As a result, the labeled amino acids and peptides are readily distinguished from unlabeled ones by the ion/mass pattern in the resulting mass spectrum. Preferably, the ion mass signature component imparts a mass to a protein fragment that does not match the residue mass for any of the 20 natural amino acids.

The label should be robust under the fragmentation conditions of MS and not undergo unfavorable fragmentation. Labeling chemistry should be efficient under a range of conditions, particularly denaturing conditions, and the labeled tag preferably remains soluble in the MS buffer system of choice. The label preferably does not suppress the ionization efficiency of the protein and is not chemically reactive. The label may contain a mixture of two or more isotopically distinct species to generate a unique mass spectrometric pattern at each labeled fragment position. Stable isotopes, such as ²H, ¹³C, ¹⁵N, ¹⁷O, ¹⁸O, or ³⁴S, are among preferred labels. Pairs of peptide internal standards that incorporate a different isotope label may also be prepared. Preferred amino acid residues into which a heavy isotope label may be incorporated include leucine, proline, valine, and phenylalanine.

Peptide internal standards are characterized according to their mass-to-charge (m/z) ratio, and preferably, also according to their retention time on a chromatographic column (e.g. an HPLC column). Internal standards that co-elute with unlabeled peptides of identical sequence are selected as optimal internal standards. The internal standard is then analyzed by fragmenting the peptide by any suitable means, for example by collision-induced dissociation (CID) using, e.g., argon or helium as a collision gas. The fragments are then analyzed, for example by multi-stage mass spectrometry (MSⁿ) to obtain a fragment ion spectrum, to obtain a peptide fragmentation signature. Preferably, peptide fragments have significant differences in m/z ratios to enable peaks corresponding to each fragment to be well separated, and a signature is that is unique for the target peptide is obtained. If a suitable fragment signature is not obtained at the first stage, additional stages of MS are performed until a unique signature is obtained.

Fragment ions in the MS/MS and MS³ spectra are typically highly specific for the peptide of interest, and, in conjunction with LC methods, allow a highly selective means of detecting and quantifying a target peptide/protein in a complex protein mixture, such as a cell lysate, containing many thousands or tens of thousands of proteins. Any biological sample potentially containing a target protein/peptide of interest may be assayed. Crude or partially purified cell extracts are preferably employed. Generally, the sample has at least 0.01 mg of protein, typically a concentration of 0.1-10 mg/mL, and may be adjusted to a desired buffer concentration and pH.

A known amount of a labeled peptide internal standard, preferably about 10 femtomoles, corresponding to a target protein to be detected/quantified is then added to a biological sample, such as a cell lysate. The spiked sample is then digested with one or more protease(s) for a suitable time period to allow digestion. A separation is then performed (e.g. by HPLC, reverse-phase HPLC, capillary electrophoresis, ion exchange chromatography, etc.) to isolate the labeled internal standard and its corresponding target peptide from other peptides in the sample. Microcapillary LC is a preferred method.

Each isolated peptide is then examined by monitoring of a selected reaction in the MS. This involves using the prior knowledge gained by the characterization of the peptide internal standard and then requiring the MS to continuously monitor a specific ion in the MS/MS or MSⁿ spectrum for both the peptide of interest and the internal standard. After elution, the area under the curve (AUC) for both peptide standard and target peptide peaks are calculated. The ratio, of the two areas provides the absolute quantification that can be normalized for the number of cells used in the analysis and the protein's molecular weight, to provide the precise number of copies of the protein per cell. Further details of the AQUA methodology are described in Gygi *et al.,* and Gerber *et al. supra*.

In accordance with the present invention, AQUA internal peptide standards (heavy-isotope labeled peptides) may now be produced, as described above, for the novel T-cell receptor signaling protein phosphorylation sites disclosed herein (see Table 1/Figure 2). Peptide standards for a given phosphorylation site may be produced for both the phosphorylated and non-phosphorylated forms of the site and such standards employed in the AQUA methodology to detect and quantify both forms of such phosphorylation site in a biological sample.

The phosphorylation site peptide sequences disclosed herein (see Column G of Table 1/Figure 2) are particularly well suited for development of corresponding AQUA peptides, since the IAP method by which they were identified (see Part A above and Example 1) inherently confirmed that such peptides are in fact produced by enzymatic digestion (trypsinization) and are in fact suitably fractionated/ionized in MS/MS. Thus, heavy-isotope labeled equivalents of these peptides (both in phosphorylated and unphosphorylated form) can be readily synthesized and their unique MS and LC-SRM signature determined, so that the peptides are validated as AQUA peptides and ready for use in quantification experiments.

Accordingly, the invention provides heavy-isotope labeled peptides (AQUA peptides) for the detection and/or quantification of any of the T-cell receptor signaling protein phosphorylation sites disclosed in rows 61 or 62 of Table 1/Figure2 (see Column G) and/or their corresponding parent protein/polypeptide (see Column A). Each such phosphorylation sequence may be considered a preferred AQUA peptide of the invention. Optimally, an AQUA peptide of the invention consists of a phosphorylation site sequence enumerated in Table 1.

However, it will be appreciated that a larger AQUA peptide comprising the disclosed phosphorylation site sequence (and additional residues downstream or upstream of it) may also be constructed. Similarly, a smaller AQUA peptide comprising less than all of the residues of a disclosed phosphorylation site sequence (but still comprising the phosphorylatable residue enumerated in Column F of Table 1/Figure 2) may alternatively be constructed. Such larger AQUA peptides are within the scope of the present invention, and the selection and production of preferred AQUA peptides may be carried out as described above (see Gygi *et al.,* Gerber *et al. supra*.).

Certain particularly preferred subsets of AQUA peptides are described above (corresponding to particular protein types/groups in Table 1, for example, Adaptor/Scaffold proteins or RNA Binding Proteins). Example 4 is provided to further illustrate the construction and use, by standard methods described above, of exemplary AQUA peptides.

AQUA peptides of the invention may also be employed within a kit that comprises one or multiple AQUA peptide(s) provided herein (for the quantification of an T-cell receptor signal transduction protein disclosed in Table 1), and, optionally, a second detecting reagent conjugated to a detectable group. For example, a kit may include AQUA peptides for both the phosphorylation and non-phosphorylated form of a phosphorylation site disclosed herein. The reagents may also include ancillary agents such as buffering agents and protein stabilizing agents, *e*.*g*., polysaccharides and the like. The kit may further include, where necessary, other members of the signal-producing system of which system the detectable group is a member (*e*.*g*., enzyme substrates), agents for reducing background interference in a test, control reagents, apparatus for conducting a test, and the like. The test kit may be packaged In any suitable manner, typically with all elements in a single container along with a sheet of printed instructions for carrying out the test.

AQUA peptides provided by the invention will be highly useful in the further study of signal transduction anomalies underlying diseases, including lymphomas, involving altered T-cell receptor signaling, and in identifying diagnostic/bio-markers of these diseases, new potential drug targets, and/or in monitoring the effects of test compounds on T-cell receptor signal transduction proteins and pathways.

### D. Immunoassay Formats

Antibodies provided by the invention may be advantageously employed in a variety of standard immunological assays (the use of AQUA peptides provided by the invention is described separately above). Assays may be homogeneous assays or heterogeneous assays. In a homogeneous assay the immunological reaction usually involves a phosphorylation-site specific antibody of the invention, a labeled analyte, and the sample of interest. The signal arising from the label is modified, directly or indirectly, upon the binding of the antibody to the labeled analyte. Both the immunological reaction and detection of the extent thereof are carried out in a homogeneous solution. Immunochemical labels that may be employed include free radicals, radioisotopes, fluorescent dyes, enzymes, bacteriophages, coenzymes, and so forth.

In a heterogeneous assay approach, the reagents are usually the specimen, a phosphorylation-site specific antibody of the invention, and suitable means for producing a detectable signal. Similar specimens as described above may be used. The antibody is generally immobilized on a support, such as a bead, plate or slide, and contacted with the specimen suspected of containing the antigen in a liquid phase. The support is then separated from the liquid phase and either the support phase or the liquid phase is examined for a detectable signal employing means for producing such signal. The signal is related to the presence of the analyte in the specimen. Means for producing a detectable signal include the use of radioactive labels, fluorescent labels, enzyme labels, and so forth. For example, if the antigen to be detected contains a second binding site, an antibody which binds to that site can be conjugated to a detectable group and added to the liquid phase reaction solution before the separation step. The presence of the detectable group on the solid support indicates the presence of the antigen in the test sample. Examples of suitable immunoassays are the radioimmunoassay, immunofluorescence methods, enzyme-linked immunoassays, and the like.

Immunoassay formats and variations thereof that may be useful for carrying out the methods disclosed herein are well known in the art. *See generally* E. Maggio, Enzyme-Immunoassay, (1980) (CRC Press, Inc., Boca Raton, Fla.); see also, *e.g*., U.S. Pat. No. 4,727,022 (Skold et al*.,* "Methods for Modulating Ligand-Receptor Interactions and their Application"); U.S. Pat. No. 4,659,678 (Forrest et al*.,* "Immunoassay of Antigens"); U.S. Pat. No. 4,376,110 (David et al*.,* "Immunometric Assays Using Monoclonal Antibodies"). Conditions suitable for the formation of reagent-antibody complexes are well described. *See id.* Monoclonal antibodies of the invention may be used in a "two-site" or "sandwich" assay, with a single cell line serving as a source for both the labeled monoclonal antibody and the bound monoclonal antibody. Such assays are described in U.S. Pat. No. 4,376,110. The concentration of detectable reagent should be sufficient such that the binding of a target T-cell receptor signal transduction protein is detectable compared to background.

Phosphorylation site-specific antibodies disclosed herein may be conjugated to a solid support suitable for a diagnostic assay (*e*.*g*., beads, plates, slides or wells formed from materials such as latex or polystyrene) in accordance with known techniques, such as precipitation. Antibodies, or other target protein or target site-binding reagents, may likewise be conjugated to detectable groups such as radiolabels (*e*.*g*., ³⁵S, ¹²⁵I, ¹³¹I), enzyme labels (*e*.*g*., horseradish peroxidase, alkaline phosphatase), and fluorescent labels (*e*.*g*., fluorescein) in accordance with known techniques.

Antibodies of the invention may also be optimized for use in a flow cytometry assay to determine the activation/phosphorylation status of a target T-cell receptor signaling protein in patients before, during, and after treatment with a drug targeted at inhibiting phosphorylation at such a protein at the phosphorylation site disclosed herein. For example, bone marrow cells or peripheral blood cells from patients may be analyzed by flow cytometry for target T-cell receptor signaling protein phosphorylation, as well as for markers identifying various hematopoietic cell types. In this manner, activation status of the malignant cells may be specifically characterized. Flow cytometry may be carried out according to standard methods. *See, e.g.* Chow et al., Cytometry (Communications in Clinical Cytometry) 46: 72-78 (2001). Briefly and by way of example, the following protocol for cytometric analysis may be employed: fixation of the cells with 1% para-formaldehyde for 10 minutes at 37°C followed by permeabilization in 90% methanol for 30 minutes on ice. Cells may then be stained with the primary antibody (a phospho-specific antibody of the invention), washed and labeled with a fluorescent-labeled secondary antibody. Alternatively, the cells may be stained with a fluorescent-labeled primary antibody. The cells would then be analyzed on a flow cytometer (*e*.*g*. a Beckman Coulter EPICS-XL) according to the specific protocols of the instrument used. Such an analysis would identify the presence of activated T-cell receptor signal transduction protein(s) in the diseased cells and reveal the drug response on the targeted protein.

Alternatively, antibodies of the invention may be employed in immunohistochemical (IHC) staining to detect differences in signal transduction or protein activity using normal and diseased tissues. IHC may be carried out according to well-known techniques. *See, e.g.,* ANTIBODIES: A LABORATORY MANUAL, supra. Briefly, paraffin-embedded tissue (*e*.*g*. tumor tissue) is prepared for immunohistochemical staining by deparaffinizing tissue sections with xylene followed by ethanol; hydrating in water then PBS; unmasking antigen by heating slide in sodium citrate buffer; incubating sections in hydrogen peroxide; blocking in blocking solution; incubating slide in primary antibody and secondary antibody; and finally detecting using ABC avidin/biotin method according to manufacturer's instructions.

Antibodies of the invention may be also be optimized for use in other clinically-suitable applications, for example bead-based multiplex-type assays, such as IGEN, Luminex^{™} and/or Bioplex^{™} assay formats, or otherwise optimized for antibody arrays formats, such as reversed-phase array applications (*see*, *e.g.* Paweletz et al., Oncogene 20(16): 1981-89 (2001)). Accordingly, in another embodiment, a method is provided for the multiplex detection of T-cell receptor signaling protein phosphorylation in a biological sample, the method comprising utilizing at two antibodies or AQUA peptides to detect the presence of two phosphorylated T-cell receptor signaling proteins enumerated in Column A of Table 1/Figure 2. In one preferred embodiment, two to five antibodies or AQUA peptides are employed in the method. In another preferred embodiment, six to ten antibodies or AQUA peptides are employed, while in another preferred embodiment eleven to twenty such reagents are employed.

Antibodies and/or AQUA peptides of the invention may also be employed within a kit that comprises at least one phosphorylation site-specific antibody or AQUA peptide of the invention (which binds to or detects an T-cell receptor signaling protein/site disclosed in Table 1), and, optionally, a second antibody conjugated to a detectable group. In some embodies, the kit is suitable for multiplex assays and comprises two or more antibodies or AQUA peptides, and in some embodiments, comprises two to five, six to ten, or eleven to twenty reagents. The kit may also include ancillary agents such as buffering agents and protein stabilizing agents, *e.g*., polysaccharides and the like. The kit may further include, where necessary, other members of the signal-producing system of which system the detectable group is a member (*e*.*g*., enzyme substrates), agents for reducing background interference in a test, control reagents, apparatus for conducting a test, and the like. The test kit may be packaged in any suitable manner, typically with all elements in a single container along with a sheet of printed instructions for carrying out the test.

### EXAMPLE 1

### Isolation of Phosphotyrosine-Containing Peptides from Extracts of Activated Jurkat Cells and Identification of Novel Phosphorylation Sites.

In order to discover previously unknown T-cell receptor signaling protein phosphorylation sites, IAP isolation techniques were employed to identify phosphotyrosine-containing peptides in cell extracts from Jurkat cells treated with pervanadate in order to stimulate tyrosine phosphorylation.

Tryptic phosphotyrosine peptides were purified and analyzed from extracts of the Jurkat cell line as follows. Cells were cultured in RPMI medium supplemented with 10% bovine serum and penicillin/streptomycin. Cells were cultured to a density of 1.2 x 10⁶ cells/ml and were washed in PBS at room temperature, then resuspended in PBS at 7 x 10⁷ cells/ml. After preincubation at 37° C for 20 min, calyculin A and sodium pervanadate were added to final concentrations of 50 ng/ml and 1 mM, respectively, and cells were incubated for 20 min at 37° C. After centrifugation at room temperature, cells were resuspended at 1.25 x 10⁸ cells/ml in lysis buffer (20 mM HEPES pH 8.0. 9 M urea, 1 mM sodium vanadate) and sonicated.

Sonicated cell lysates were cleared by centrifugation at 20,000 x g, and proteins were reduced with DTT at a final concentration of 4.1 mM and alkylated with iodoacetamide at 8.3 mM. For digestion with trypsin, protein extracts were diluted in 20 mM HEPES pH 8.0 to a final concentration of 2 M urea and immobilized TLCK-trypsin (Pierce) was added at 1-2.5 ml beads (200 TAME units trypsin/ml) per 10⁹ cells. Digestion was performed for 1-2 days at room temperature.

Trifluoroacetic acid (TFA) was added to protein digests to a final concentration of 1%, precipitate was removed by centrifugation, and digests were loaded onto Sep-Pak C₁₈ columns (Waters) equilibrated with 0.1 % TFA. A column volume of 0.7-1.0 ml was used per 2 x 10⁸ cells. Columns were washed with 15 volumes of 0.1 % TFA, followed by 4 volumes of 5% acetonitrile (MeCN) in 0.1% TFA. Peptide fraction I was obtained by eluting columns with 2 volumes each of 8, 12, and 15% MeCN in 0.1% TFA and combining the eluates. Fractions II and III were a combination of eluates after eluting columns with 18, 22, 25% MeCN in 0.1% TFA and with 30, 35, 40% MeCN in 0.1% TFA, respectively. All peptide fractions were lyophilized.

Peptides from each fraction corresponding to 2 x 10⁸ cells were dissolved in 1 ml of IAP buffer (20 mM Tris/HCl or 50 mM MOPS pH 7.2, 10 mM sodium phosphate, 50 mM NaCl) and insoluble matter (mainly in peptide fractions III) was removed by centrifugation. IAP was performed on each peptide fraction separately. The phosphotyrosine monoclonal antibody P-Tyr-100 (Cell Signaling Technology, Inc., catalog number 9411) was coupled at 4 mg/ml beads to protein G agarose (Roche).

Immobilized antibody (15 µl, 60 µg) was added as 1:1 slurry in lAP buffer to 1 ml of each peptide fraction, and the mixture was incubated overnight at 4° C with gentle rotation. The immobilized antibody beads were washed three times with 1 ml IAP buffer and twice with 1 ml water, all at 4° C. Peptides were eluted from beads by incubation with 75 µl of 0.1 % TFA at room temperature for 10 min.

### Analysis by MALDI-TOF Mass Spectrometry.

A thin layer of a-cyano-4-hydroxy-cinnamic acid (ACHA) matrix was applied to a Bruker 384-spot MALDI target by spreading 5 µl of a saturated solution in MeCN/water (2/1, v/v) over an entire row of spots on the target; drying occurred in 2-5 sec. The lAP eluate (10 µl) was loaded onto an 0.2 µl C-18 ZipTip (Millipore), which then was washed with 5% formic acid. Peptide was eluted with 1 µl of 10 mg/ml ACHA in 60% methanol, 5% formic acid onto the MALDI target containing the thin layer of matrix. Samples were analyzed on a Bruker BiFlex III MALDI-TOF instrument in positive ion mode.

### Analysis by LC-MS/MS Mass Spectrometry.

40 µl of IAP eluate were purified by 0.2 µl C-18 ZipTip (Millipore). Peptides were eluted from the microcolumns with 1 µl of 40% MeCN, 0.1% TFA (fractions I and II) or 1 µl of 60% MeCN, 0.1% TFA (fraction III) into 7.6 µl of 0.4% acetic acid/0.005% heptafluorobutyric acid. This sample was loaded onto a 10 cm x 75 µm PicoFrit capillary column (New Objective) packed with Magic C18 AQ reversed-phase resin (Michrom Bioresources) using a Famos autosampler with an inert sample injection valve (Dionex). The column was then developed with a 45-min linear gradient of acetonitrile delivered at 200 nl/min (Ultimate, Dionex), and tandem mass spectra were collected in a data-dependent manner with an LCQ Deca XP Plus ion trap mass spectrometer essentially as described by Gygi *et al., supra*.

### Database Analysis & Assignments.

MS/MS spectra were evaluated using TurboSequest in the Sequest Browser package (v. 27, rev. 12) supplied as part of BioWorks 3.0 (ThermoFinnigan). Individual MS/MS spectra were extracted from the raw data file using the Sequest Browser program CreateDta, with the following settings: bottom MW, 700; top MW, 4,500; minimum number of ions, 20; minimum TIC, 4x10⁵; and precursor charge state, unspecified. Spectra were extracted from the beginning of the raw data file before sample injection to the end of the eluting gradient. The lonQuest and VuDta programs were not used to further select MS/MS spectra for Sequest analysis. MS/MS spectra were evaluated with the following TurboSequest parameters: peptide mass tolerance, 2.5; fragment ion tolerance, 0.0; maximum number of differential amino acids per modification, 4; mass type parent, average; mass type fragment, average; maximum number of internal cleavage sites, 10; neutral losses of water and ammonia from b and y ions were considered in the correlation analysis. Proteolytic enzyme was specified except for spectra collected from elastase digests.

Searches were performed against the NCBI human protein database (released on April 29, 2003 and containing 37,490 protein sequences). Cysteine carboxamidomethylation was specified as a static modification, and phosphorylation was allowed as a variable modification on serine, threonine, and tyrosine residues or on tyrosine residues alone. It was determined that restricting phosphorylation to tyrosine residues had little effect on the number of phosphorylation sites assigned.

In proteomics, it is desirable to validate protein identifications based solely on the observation of a single peptide in one experimental result, in order to indicate that the protein is, in fact, present in a sample. This has led to the development of statistical methods for validating peptide assignments, which are not yet universally accepted, and guidelines for the publication of protein and peptide identification results (*see* Carr et al. Mol Cell Proteomics 3: 531-533 (2004), which were followed in this Example. However, because the immunoaffinity strategy separates phosphorylated peptides from unphosphorylated peptides, observing just one phosphopeptide from a protein is a common result, since many phosphorylated proteins have only one tyrosine-phosphorylated site. For this reason, it is appropriate to use additional criteria to validate phosphopeptide assignments. Assignments are likely to be correct if any of these additional criteria are met: (i) the same sequence is assigned to co-eluting ions with different charge states, since the MS/MS spectrum changes markedly with charge state; (ii) the site is found in more than one peptide sequence context due to sequence overlaps from incomplete proteolysis or use of proteases other than trypsin; (iii) the site is found in more than one peptide sequence context due to homologous but not identical protein isoforms; (iv) the site is found in more than one peptide sequence context due to homologous but not identical proteins among species; and (v) sites validated by MS/MS analysis of synthetic phosphopeptides corresponding to assigned sequences, since the ion trap mass spectrometer produces highly reproducible MS/MS spectra. The last criterion is routinely employed to confirm novel site assignments of particular interest.

All spectra and all sequence assignments made by Sequest were imported into a relational database. Assigned sequences were accepted or rejected following a conservative, two-step process. In the first step, a subset of high-scoring sequence assignments was selected by filtering for XCorr values of at least 1.5 for a charge state of +1, 2.2 for +2, and 3.3 for +3, allowing a maximum RSp value of 10. Assignments in this subset were rejected if any of the following criteria were satisfied: (i) the spectrum contained at least one major peak (at least 10% as intense as the most intense ion in the spectrum) that could not be mapped to the assigned sequence as an *a*, *b*, or *y* ion, as an ion arising from neutral-loss of water or ammonia from a *b* or *y* ion, or as a multiply protonated ion; (ii) the spectrum did not contain an series of *b* or *y* ions equivalent to at least six uninterrupted residues; or (iii) the sequence was not observed at least five times in all the studies we have conducted (except for overlapping sequences due to incomplete proteolysis or use of proteases oth er than trypsin). In the second step, assignments with below-threshold scores were accepted if the low-scoring spectrum showed a high degree of similarity to a high-scoring spectrum collected in another study, which simulates a true reference library-searching strategy. All spectra supporting the final list of 95 assigned sequences enumerated in Table 1/Figure 2 herein were reviewed by at least three people to establish their credibility.

### EXAMPLE 2

### Production of Phospho-specific Polyclonal Antibodies for the Detection of T-cell receptor Signaling Protein Phosphorylation

Polyclonal antibodies that specifically bind a T-cell receptor signal transduction protein only when phosphorylated at the respective phosphorylation site disclosed herein (see Table 1) are produced according to standard methods by first constructing a synthetic peptide antigen comprising the phosphorylation site sequence and then immunizing an animal to raise antibodies against the antigen, as further described below. Production of exemplary polyclonal antibodies is provided below.

### A. Cdk6 (tyrosine 24).

A 15 amino acid phospho-peptide antigen, AEIGEGAy*GKVFKAR (SEQ ID NO: 61) (where y*= phosphotyrosine), that corresponds to the tyrosine 24 phosphorylation site in human Cdk6 kinase (see Row 62 of Table 1), plus cysteine on the C-terminal for coupling, is constructed according to standard synthesis techniques using, *e*.*g*., a Rainin/Protein Technologies, Inc., Symphony peptide synthesizer. See ANTIBODIES: A LABORATORY MANUAL, *supra.;* Merrifield, *supra.* This peptide is then coupled to KLH and used to immunize animals to produce (and subsequently screen) phospho-specific Cdk6 (Tyr24) polyclonal antibodies as described in Immunization/ Screening below.

### B. ZAP70 (tyrosine 248).

A 15 amino acid phospho-peptide antigen, LKADGLIy*CLKEACP (SEQ ID NO: 63) (where y*= phosphotyrosine), that corresponds to the tyrosine 248 phosphorylation site in human ZAP70 kinase (see Row 64 of Table 1), plus cysteine on the C-terminal for coupling, is constructed according to standard synthesis techniques using, *e*.*g*., a Rainin/Protein Technologies, Inc., Symphony peptide synthesizer. *See* ANTIBODIES: A LABORATORY MANUAL, *supra.;* Merrifield, *supra.* This peptide is then coupled to KLH and used to immunize animals to produce (and subsequently screen) phospho-specific ZAP70 (Tyr248) polyclonal antibodies as described in Immunization/Screening below.

### C. SIT (tyrosine 95).

A 15 amino acid phospho-peptide antigen, PLYGNLHy*LQTGRLS (SEQ ID NO: 12) (where y*= phosphotyrosine) that corresponds to the tyrosine 95 phosphorylation site in human SIT protein (see Row 13 of Table 1), plus cysteine on the C-terminal for coupling, is constructed according to standard synthesis techniques using, *e*.*g*., a Rainin/Protein Technologies, Inc., Symphony peptide synthesizer. *See* ANTIBODIES: A LABORATORY MANUAL, *supra.;* Merrifield, *supra.* This peptide is then coupled to KLH and used to immunize animals to produce (and subsequently screen) phospho-specific SIT (Tyr95) antibodies as described in Immunization/Screening below.

### Immunization/Screening.

A synthetic phospho-peptide antigen as described in A-C above is coupled to KLH, and rabbits are injected intradermally (ID) on the back with antigen in complete Freunds adjuvant (500 µg antigen per rabbit). The rabbits are boosted with same antigen in incomplete Freund adjuvant (250 µg antigen per rabbit) every three weeks. After the fifth boost, bleeds are collected. The sera are purified by Protein A-affinity chromatography by standard methods (see ANTIBODIES: A LABORATORY MANUAL, Cold Spring Harbor, *supra*.). The eluted immunoglobulins are further loaded onto a non-phosphorylated synthetic peptide antigen-resin Knotes column to pull out antibodies that bind the non-phosphorylated form of the phosphorylation site. The flow through fraction is collected and applied onto a phospho-synthetic peptide antigen-resin column to isolate antibodies that bind the phosphorylated form of the site. After washing the column extensively, the bound antibodies (*i*.*e*. antibodies that bind a phosphorylated peptide described in A-C above, but do not bind the non-phosphorylated form of the peptide, are eluted and kept in antibody storage buffer.

The isolated antibody is then tested for phospho-specificity using Western blot assay using an appropriate cell line the expresses (or overexpresses) target phospho-protein (*i*.*e*. phosphorylated Cdk6, ZAP70, or SIT), for example, Jurkat cells. Cells are cultured in RPMI medium supplemented with 10% FCS and penicillin/streptomycin. Before stimulation, the cells are starved in serum-free RPMI medium for 4 hours. The cells are then stimulated with ligand (*e*.*g*. 50 ng/ml) for 5 minutes. Cell are collected, washed with PBS and directly lysed in cell lysis buffer. The protein concentration of cell lysates are then measured. The loading buffer is added into cell lysate and the mixture is boiled at 100°C for 5 minutes. 20 µl (10 µg protein) of sample is then added onto 7.5% SDS-PAGE gel.

A standard Western blot may be performed according to the lmmunoblotting Protocol set out in the CELL SIGNALING TECHNOLOGY, INC. 2003-04 Catalogue, p. 390. The isolated phospho-specific antibody is used at dilution 1:1000. Phosphorylation-site specificity of the antibody will be shown by binding of only the phosphorylated form of the target protein. Isolated phospho-specific polyclonal antibody does not recognize the target protein when not phosphorylated at the appropriate phosphorylation site in the non-stimulated cells (*e.g*. ZAP70 is not bound when not phosphorylated at tyrosine 248).

In order to confirm the specificity of the isolated antibody, different cell lysates containing various phosphorylated signal transduction proteins other than the target protein are prepared. The Western blot assay is preformed again using these cell lysates. The phospho-specific polyclonal antibody isolated as described above is used (1:1000 dilution) to test reactivity with the different phosphorylated non-target proteins on Western blot membrane. The phospho-specific antibody does not significantly cross-react with other phosphorylated signal transduction proteins, although occasionally slight binding with a highly-homologous phosphorylation-site on another protein may be observed. In such case the antibody may be further purified using affinity chromatography, or the specific immunoreactivity cloned by rabbit hybridoma technology.

### EXAMPLE 3

### Production of Phospho-specific Monoclonal Antibodies for the Detection of T-cell Receptor Signaling Protein Phosphorylation

Monoclonal antibodies that specifically bind a T-cell receptor signal transduction protein only when phosphorylated at the respective phosphorylation site disclosed herein (see Table 1) are produced according to standard methods by first constructing a synthetic peptide antigen comprising the phosphorylation site sequence and then immunizing an animal to raise antibodies against the antigen, and harvesting spleen cells from such animals to produce fusion hybridomas, as further described below. Production of exemplary monoclonal antibodies is provided below.

### A. Cdk6 (tyrosine 13).

A 15 amino acid phospho-peptide antigen, LCRADQQy*ECVAEIG (SEQ ID NO: 60) (where y*= phosphotyrosine) that corresponds to the tyrosine 13 phosphorylation site in human Cdk6 kinase (see Row 61 of Table 1), plus cysteine on the C-terminal for coupling, is constructed according to standard synthesis techniques using, *e*.*g*., a Rainin/Protein Technologies, Inc., Symphony peptide synthesizer. *See* ANTIBODIES: A LABORATORY MANUAL, *supra.;* Merrifield, *supra.* This peptide is then coupled to KLH and used to immunize animals and harvest spleen cells for generation (and subsequent screening) of phospho-specific monoclonal Cdk6 (Tyr13) antibodies as described in Immunization/Fusion/Screening below.

### B. FAF-X (tyrosine 2533).

A 15 amino acid phospho-peptide antigen, GQRAQENy*EGSEEVS (SEQ ID NO: 59) (where y*= phosphotyrosine) that corresponds to the tyrosine 2533 phosphorylation site in human FAF-X protease (see Row 60 of Table 1), plus cysteine on the C-terminal for coupling, is constructed according to standard synthesis techniques using, *e*.*g*., a Rainin/Protein Technologies, Inc., Symphony peptide synthesizer. *See* ANTIBODIES: A LABORATORY MANUAL, *supra.;* Merrifield, *supra.* This peptide is then coupled to KLH and used to immunize animals and harvest spleen cells for generation (and subsequent screening) of phospho-specific monoclonal FAF-X (Tyr2533) antibodies as described in Immunization/Fusion/Screening below.

### C. Cortactin-a (tyrosine 453).

A 15 amino acid phospho-peptide antigen, YSMEAADy*REASSQQ (SEQ ID NO: 43) (where y*= phosphotyrosine) that corresponds to the tyrosine 453 phosphorylation site in human Cortacin (isoform a) protein (see Row 44 of Table 1), plus cysteine on the C-terminal for coupling, is constructed according to standard synthesis techniques using, *e*.*g*., a Rainin/Protein Technologies, Inc., Symphony peptide synthesizer. *See* ANTIBODIES: A LABORATORY MANUAL, *supra.;* Merrifield, *supra.* This peptide is then coupled to KLH and used to immunize animals and harvest spleen cells for generation (and subsequent screening) of phospho-specific monoclonal Cortactin-a (Tyr453) antibodies as described in Immunization/ Fusion/Screening below.

### Immunization/Fusion/Screening.

A synthetic phospho-peptide antigen as described in A-C above is coupled to KLH, and BALB/C mice are injected intradermally (ID) on the back with antigen in complete Freunds adjuvant (*e*.*g*. 50 µg antigen per mouse). The mice are boosted with same antigen in incomplete Freund adjuvant (*e*.*g*. 25 µg antigen per mouse) every three weeks. After the fifth boost, the animals are sacrificed and spleens are harvested.

Harvested spleen cells are fused to SP2/0 mouse myeloma fusion partner cells according to the standard protocol of Kohler and Milstein (1975). Colonies originating from the fusion are screened by ELISA for reactivity to the phospho-peptide and non-phospho-peptide forms of the antigen and by Western blot analysis (as described in Example 1 above). Colonies found to be positive by ELISA to the phospho-peptide while negative to the non-phospho-peptide are further characterized by Western blot analysis. Colonies found to be positive by Western blot analysis are subcloned by limited dilution. Mouse ascites are produced from a single clone obtained from subcloning, and tested for phospho-specificity (against the Cdk6, FAF-X, or Cortactin-a phospho-peptide antigen, as the case may be) on ELISA. Clones identified as positive on Western blot analysis using cell culture supernatant as having phospho-specificity, as indicated by a strong band in the induced lane and a weak band in the uninduced lane of the blot, are isolated and subcloned as clones producing monoclonal antibodies with the desired specificity.

Ascites fluid from isolated clones may be further tested by Western blot analysis. The ascites fluid should produce similar results on Western blot analysis as observed previously with the cell culture supernatant, indicating phospho-specificity against the phosphorylated target (*e*.*g*. FAF-X phosphorylated at tyrosine 2533).

### EXAMPLE 4

### Production and Use of AQUA Peptides for the Quantification of T-cell Receptor Signaling Protein Phosphorylation

Heavy-isotope labeled peptides (AQUA peptides (internal standards)) for the detection and quantification of an T-cell receptor signal transduction protein only when phosphorylated at the respective phosphorylation site disclosed herein (*see* Table 1) are produced according to the standard AQUA methodology (*see* Gygi *et al.,* Gerber *et al., supra*.) methods by first constructing a synthetic peptide standard corresponding to the phosphorylation site sequence and incorporating a heavy-isotope label. Subsequently, the MSⁿ and LC-SRM signature of the peptide standard is validated, and the AQUA peptide is used to quantify native peptide in a biological sample, such as a digested cell extract. Production and use of exemplary AQUA peptides is provided below.

### A. LPP (tyrosine 317).

An AQUA peptide having a sequence corresponding to the tyrosine 317 phosphorylation site in human Lipoma-preferred-partner (LPP) protein, RNDSDPTy*GQQGHPN (y*= phosphotyrosine) (see Row 14 in Table 1 (SEQ ID NO: 13)) but incorporating ¹⁴C/¹⁵N-labeled proline (indicated by bold **P**) is constructed according to standard synthesis techniques using, *e*.*g*., a Rainin/Protein Technologies, Inc., Symphony peptide synthesizer (*see* Merrifield, *supra*.) as further described below in Synthesis & MS/MS Signature. The LPP (Tyr317) AQUA peptide is then spiked into a biological sample to quantify the amount of phosphorylated LPP (Tyr317) in the sample, as further described below in Analysis & Quantification.

### B. Ets-1 (tyrosine 205).

An AQUA peptide having a sequence corresponding to the tyrosine 205 phosphorylation site in human Ets-1 transcription factor protein, SLKYENDy*PSVILRD (y*= phosphotyrosine) (see Row 78 in Table 1 (SEQ ID NO: 77)) but incorporating ¹⁴C/¹⁵N-labeled leucine (indicated by bold L) is constructed according to standard synthesis techniques using, *e*.*g*., a Rainin/Protein Technologies, Inc., Symphony peptide synthesizer (see Merrifield, *supra.)* as further described below in Synthesis & MS/MS Signature. The Ets-1 (Tyr205) AQUA peptide is then spiked into a biological sample to quantify the amount of phosphorylated Ets-1 (Tyr205) in the sample, as further described below in Analysis & Quantification.

### C. Bid (tyrosine 54).

An AQUA peptide having a sequence corresponding to the tyrosine 54 phosphorylation site in human Bid protein, LAPQWEGy*DELQTDG (y*= phosphotyrosine) (see Row 18 in Table 1 (SEQ ID NO: 17)) but incorporating ¹⁴C/¹⁵N-labeled leucine (indicated by bold L) is constructed according to standard synthesis techniques using, *e*.*g*., a Rainin/Protein Technologies, Inc., Symphony peptide synthesizer (*see* Merrifield, *supra*.) as further described below in Synthesis & MS/MS Signature. The Bid (Tyr54) AQUA peptide is then spiked into a biological sample to quantify the amount of phosphorylated Bid (Tyr54) in the sample, as further described below in Analysis & Quantification.

### D. GIT2 (tyrosine 492).

An AQUA peptide having a sequence corresponding to the tyrosine 492 phosphorylation site in human GIT2 protein, QVQTGSEy*TDTSNHS (y*= phosphotyrosine) (see Row 51 in Table 1 (SEQ ID NO: 50)) but incorporating ¹⁴C/¹⁵N-labeled valine (indicated by bold **V**) is constructed according to standard synthesis techniques using, *e*.*g*., a Rainin/Protein Technologies, Inc., Symphony peptide synthesizer (*see* Merrifield, *supra*.) as further described below in Synthesis & MS/MS Signature. The GIT2 (Tyr492) AQUA peptide is then spiked into a biological sample to quantify the amount of phosphorylated GIT2 (Tyr492) in the sample, as further described below in Analysis & Quantification.

### Synthesis & MS/MS Spectra.

Fluorenylmethoxycarbonyl (Fmoc)-derivatized amino acid monomers may be obtained from AnaSpec (San Jose, CA). Fmoc-derivatized stable-isotope monomers containing one ¹⁵N and five to nine ¹³C atoms may be obtained from Cambridge Isotope Laboratories (Andover, MA). Preloaded Wang resins may be obtained from Applied Biosystems. Synthesis scales may vary from 5 to 25 µmol. Amino acids are activated *in situ* with 1-H-benzotriazolium, 1-bis(dimethylamino) methylene]-hexafluorophosphate(1-),3-oxide:1-hydroxybenzotriazole hydrate and coupled at a 5-fold molar excess over peptide. Each coupling cycle is followed by capping with acetic anhydride to avoid accumulation of one-residue deletion peptide byproducts. After synthesis peptide-resins are treated with a standard scavenger-containing trifluoroacetic acid (TFA)-water cleavage solution, and the peptides are precipitated by addition to cold ether. Peptides (*i*.*e*. a desired AQUA peptide described in A-D above) are purified by reversed-phase C18 HPLC using standard TFA/acetonitrile gradients and characterized by matrix-assisted laser desorption ionization-time of flight (Biflex III, Bruker Daltonics, Billerica, MA) and ion-trap (ThermoFinnigan, LCQ DecaXP) MS.

MS/MS spectra for each AQUA peptide should exhibit a strong y-type ion peak as the most intense fragment ion that is suitable for use in an SRM monitoring/analysis. Reverse-phase microcapillary columns (0.1 A- 150-220 mm) are prepared according to standard methods. An Agilent 1100 liquid chromatograph may be used to develop and deliver a solvent gradient [0.4% acetic acid/0.005% heptafluorobutyric acid (HFBA)/7% methanol and 0.4% acetic acid/0.005% HFBA/65% methanol/35% acetonitrile] to the microcapillary column by means of a flow splitter. Samples are then directly loaded onto the microcapillary column by using a FAMOS inert capillary autosampler (LC Packings, San Francisco) after the flow split. Peptides are reconstituted in 6% acetic acid/0.01% TFA before injection.

### Analysis & Quantification.

Target protein (*e*.*g*. a phosphorylated protein of A-D above) in a biological sample is quantified using a validated AQUA peptide (as described above). The IAP method is then applied to the complex mixture of peptides derived from proteolytic cleavage of crude cell extracts to which the AQUA peptides have been spiked in.

LC-SRM of the entire sample is then carried out. MS/MS may be performed by using a ThermoFinnigan (San Jose, CA) mass spectrometer (LCQ DecaXP ion trap or TSQ Quantum triple quadrupole). On the DecaXP, parent ions are isolated at 1.6 m/z width, the ion injection time being limited to 150 ms per microscan, with two microscans per peptide averaged, and with an AGC setting of 1 x 10⁸; on the Quantum, Q1 is kept at 0.4 and Q3 at 0.8 m/z with a scan time of 200 ms per peptide. On both instruments, analyte and internal standard are analyzed in alternation within a previously known reverse-phase retention window; well-resolved pairs of internal standard and analyte are analyzed in separate retention segments to improve duty cycle. Data are processed by integrating the appropriate peaks in an extracted ion chromatogram (60.15 m/z from the fragment monitored) for the native and internal standard, followed by calculation of the ratio of peak areas multiplied by the absolute amount of internal standard (*e*.*g*., 500 fmol).

## Claims

1. A method for detecting or quantifying a signaling protein that is tyrosine-phopsphorylated in T-cell receptor signaling pathways, said method comprising the step of utilizing one or more of the following reagents to detect or quantify the T-cell receptor signaling protein Cdk 6 only when phosphorylated at the tyrosine 13 or 24 :
(i) an isolated phosphorylation site-specific antibody that specifically binds said protein only when phosphorylated at the tyrosine 13 or 24, comprised within the phosphorylation site sequence SEQ ID NO: 60 or SEQ ID NO: 61 , wherein said antibody does not bind said protein when not phosphorylated at said tyrosine; and/or
(ii) a heavy-isotope labeled peptide (AQUA peptide) for the quantification of said protein, said labeled peptide comprising the phosphorylation site sequence SEQ ID NO: 60 or SEQ ID NO: 61, comprising the phosphorylated tyrosine 13 or 24

2. The method of claim 1, wherein
(i) said antibody specifically binds Cdk 6 only when phosphorylated at tyrosine 24, comprised within the phosphorylation site sequence SEQ ID NO: 61; and
(ii) said labeled peptide comprises the phosphorylation site sequence SEQ ID NO: 61, comprising the phosphorylated tyrosine 24.

3. An isolated phosphorylation site-specific antibody that specifically binds the human T-cell receptor signaling protein Cdk 6 only when phosphorylated at the tyrosine 13 or 24 comprised within the phosphorylatable peptide sequence SEQ ID NOs: 60 and 61, wherein said antibody does not bind said signaling protein when not phosphorylated at said tyrosine.

4. The antibody of claim 3, wherein said antibody specifically binds Cdk 6 only when phosphorylated at tyrosine 24 , comprised within the phosphorylatable peptide sequence SEQ ID NO: 61, wherein said antibody does not bind said protein when not phosphorylated at said tyrosine.

5. An immortalized cell line producing the antibody of claim 3 or claim 4.

6. The cell line of claim 5, wherein said immortalized cell line is a rabbit hybridoma or a mouse hybridoma.

7. A heavy-isotope labeled peptide (AQUA peptide) for the quantification of the human T-cell receptor signaling protein Cdk 6 , said labeled peptide comprising the phosphorylatable peptide sequence SEQ ID NO: 60 or SEQ ID NO: 61, which sequence comprises the phosphorylatable tyrosine 13 or 24.

8. A heavy-isotope labeled peptide of claim 7, wherein said labelled peptide comprises the phosphorylatable peptide sequence SEQ ID NO: 61, which sequence comprises the phosphorylatable tyrosine 24.

## Patentansprüche

1. Verfahren zum Nachweisen oder Quantifizieren eines Signalproteins, das in T-Zellrezeptor-Signalwegen Tyrosin-phosphoryliert wird, wobei das Verfahren den Schritt des Verwendens von einem oder mehreren der folgenden Reagenzien umfaßt, um das T-Zellrezeptor-Signalprotein Gdk6, nur wenn es am Tyrosin 13 oder 24 phosphoryliert ist, nachzuweisen oder zu quantifizieren:
(i) Ein isolierter Phosphorylierungsstellen-spezifischer Antikörper, der das Protein nur spezifisch bindet, wenn es am Tyrosin 13 oder 24, die in der Phosphorylierungsstellen-Sequenz SEQ ID NR: 60 oder SEQ ID NR: 61 umfaßt sind, phosphoryliert ist, wobei der Antikörper das Protein nicht bindet, wenn es an dem Tyrosin nicht phosphoryliert ist, und/oder
(ii) ein mit einem schweren Isotop markiertes Peptid (AQUA Peptid) für die Quantifizierung des Proteins, wobei das markierte Peptid die Phosphorylierungsstellen-Sequenz SEQ ID NR: 60 oder SEQ ID NR: 61 umfaßt, welche das phosphorylierte Tyrosin 13 oder 24 umfassen.

2. Verfahren nach Anspruch 1, wobei
(i) der Antikörper Cdk6 nur spezifisch bindet, wenn es am Tyrosin 24, das in der Phosphorylierungsstellen-Sequenz SEQ ID NR: 61 umfaßt ist, phosphoryliert ist, und
(ii) das markierte Peptid die Phosphorylierungsstellen-Sequenz SEQ ID NR: 61 umfaßt, die das phosphorylierte Tyrosin 24 umfaßt.

3. isolierter Phosphorylierungsstellen-spezifischer Antikörper, der das humane T-Zellrezeptor-Signalprotein Cdk6 nur spezifisch bindet, wenn es am Tyrosin 13 oder 24, die in der phosphorylierbaren Peptidsequenz SEQ ID NR: 60 und 61 umfaßt sind, phosphoryliert ist, wobei der Antikörper das Signalprotein nicht bindet, wenn es an dem Tyrosin nicht phosphoryliert ist.

4. Antikörper nach Anspruch 3, wobei der Antikörper Cdk6 nur spezifisch bindet, wenn es am Tyrosin 24, das in der phosphorylierbaren Peptidsequenz SEQ ID NR: 61 umfaßt ist, phosphoryliert ist, wobei der Antikörper das Protein nicht bindet, wenn es an dem Tyrosin nicht phosphoryliert ist.

5. lmmortalisierte Zelllinie, die den Antikörper nach Anspruch 3 oder Anspruch 4 herstellt.

6. Zellinie nach Anspruch 5, wobei die immortalisierte Zelllinie ein Kaninchen-Hybridom oder ein Maus-Hybridom ist.

7. Mit einem schweren Isotop markiertes Peptid (AQUA Peptid) für die Quantifizierung des humanen T-Zeiirezeptor-Signalproteins Cdk6, wobei das markierte Peptid die phosphorylierbare Peptidsequenz SEQ ID NR: 60 oder SEQ ID NR: 61 umfaßt, wobei die Sequenz das phosphorylierbare Tyrosin 13 oder 24 umfaßt.

8. Mit einem schweren Isotop markiertes Peptid nach Anspruch 7, wobei das markierte Peptid die phosphorylierbare Peptidsequenz SEQ ID NR: 61 umfaßt, wobei die Sequenz das phosphorylierbare Tyrosin 24 umfaßt.

## Revendications

1. Procédé de detection ou de quantification d'une protéine de signalisation qui est phosphorylée au niveau de la tyrosine dans les voies de signalisation des récepteurs des cellules T, ledit procédé comprenant l'étape consistant à utiliser un ou plusieurs des réactifs suivants pour détecter ou quantifier la protéine de signalisation des récepteurs des cellules T CdK6 uniquement lorsqu'elle est phosphorylée au niveau de la tyrosine 13 ou 24 :
(i) un anticorps isolé spécifique du site de phosphorylation qui se lie spécifiquement à ladite protéine uniquement lorsqu'elle est phospho-rylée au niveau de la tyrosine 13 ou 24, comprise à l'intérieur de la séquence du site de phosphorylation SEQ ID NO : 60 ou SEQ ID NO : 61, ledit anticorps ne se liant pas à ladite protéine lorsqu'elle n'est pas phosphorylée au niveau de ladite tyrosine ; et/ou
(ii) un peptide marqué par un isotope lourd (peptide AQUA) pour la quantification de ladite protéine, ledit peptide marqué comprenant la séquence du site de phosphorylation SEQ ID NO : 60 ou SEQ ID NO : 61, comprenant la tyrosine phosphorylée 13 ou 24.

2. Procédé selon la revendication 1, dans lequel :
(i) ledit anticorps se lie spécifiquement à CdK6 uniquement lorsqu'elle est phosphorylée au niveau de la tyrosine 24, comprise à l'intérieur de la séquence du site de phosphorylation SEQ ID NO : 61; et
(ii) ledit peptide marqué comprend la séquence du site de phosphorylation SEQ ID NO : 61, comprenant la tyrosine 24 phosphorylée.

3. Anticorps isolé spécifique du site de phosphorylation qui se lie spécifiquement à la protéine humaine de signalisation des récepteurs des cellules T CdK6 uniquement lorsqu'elle est phosphorylée au niveau de la tyrosine 13 ou 24 comprise à l'intérieur des séquences du peptide phosphorylable SEQ ID NO : 60 et 61, ledit anticorps ne se liant pas à ladite protéine de signalisation lorsqu'elle n'est pas phosphorylée au niveau de ladite tyrosine.

4. Anticorps selon la revendication 3, dans lequel ledit anticorps se lie spécifiquement à CdK6 uniquement lorsqu'elle est phosphorylée au niveau de la tyrosine 24, comprise à l'intérieur de la séquence du peptide phosphorylable SEQ ID NO : 61, ledit anticorps ne se liant pas à ladite protéine lorsqu'elle n'est pas phosphorylée au niveau de ladite tyrosine.

5. Lignée cellulaire immortalisée produisant l'anticorps selon la revendication 3 ou la revendication 4.

6. Lignée cellulaire selon la revendication 5, dans laquelle ladite lignée cellulaire immortalisée est un hybridome de lapin ou un hybridome de souris.

7. Peptide marqué par un isotope lourd (peptide AQUA) pour la quantification de la protéine humaine de signalisation des récepteurs des cellules T CdK6, ledit peptide marqué comprenant la séquence du peptide phosphorylable SEQ ID NO : 60 ou SEQ ID NO : 61, laquelle séquence comprend la tyrosine 13 ou 24 phosphorylable.

8. Peptide marqué par un isotope lourd selon la revendication 7, **caractérisé en ce que** peptide marqué comprend la séquence du peptide phosphorylable SEQ ID NO : 61, laquelle séquence comprend la tyrosine 24 phosphorylable.


### SEQUENCE LISTING

<110> CELL SIGNALING TECHNOLOGY, INC.
   Moritz, Albrecht
   Rush, John
   Polakiewicz, Roberto
   Lee, Kimberly
<120> REAGENTS FOR THE DETECTION OF PROTEIN PHOSPHORYLATION IN T-CELL RECEPTOR S IGNALING PATHWAYS
<130> CST-217 PCT
<160> 95
<170> PatentIn version 3.1
<210> 1
   <211> 15
   <212> PRT
   <213> Homo sapiens
<220>
   <221> MOD_RES
   <222> (8)..(8)
   <223> PHOSPHORYLATION: tyrosine at position 8 is phosphorylated.
<400> 1
   Ile Pro Lys Val Lys Ala Ile Tyr Asp Ile Glu Arg Pro Asp Leu
   1               5                   10                  15
<210> 2
   <211> 15
   <212> PRT
   <213> Homo sapiens
<220>
   <221> MOD RES
   <222> (8)..(8)
   <223> PHOSPHORYLATION: tyrosine at position 8 is phosphorylated
<400> 2
   Glu Arg Pro Asp Leu Ile Thr Tyr Glu Pro Phe Tyr Thr Ser Gly
   1               5                   10                  15
<210> 3
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 3
   Leu Ile Thr Tyr Glu Pro Phe Tyr Thr Ser Gly Tyr Asp Asp Lys
   1               5                   10                  15
<210> 4
   <211> 15
   <212> PRT
   <213> Homo sapiens
<220>
   <221> MOD_RES
   <222> (8).. (8)
   <223> PHOSPHORYLATION: tyrosine at position 8 is phosphorylated
<400> 4
   Lys Ala Pro Pro Pro Val Phe Tyr Asn Lys Pro Pro Glu Ile Asp
   1               5                   10                  15
<210> 5
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 5
   Gln Ala Pro Val Gly Ser Val Tyr Gln Lys Thr Asn Ala Val Ser
   1               5                   10                  15
<210> 6
   <211> 15
   <212> PRT
   <213> Homo sapiens
<220>
   <221> MOD_RES
   <222> (8)..(8)
   <223> PHOSPHORYLATION: tyrosine at posiiton 8 is phosphorylated
<400> 6
   Pro Tyr Glu Val Glu Val Thr Tyr Asp Gly Val Pro Val Pro Gly
   1               5                   10                  15
<210> 7
   <211> 15
   <212> PRT
   <213> Homo sapiens
<220>
   <221> MOD_RES
   <222> (8).. (8)
   <223> PHOSPHORYLATION: tyrosine at position 8 is phosphorylated
<400> 7
   Glu Pro Pro His Pro Leu Thr Tyr Ser Gln Leu Pro Arg Val Gly
   1               5                   10                  15
<210> 8
   <211> 15
   <212> PRT
   <213> Homo sapiens
<220>
   <221> MOD_RES
   <222> (8) ..(8)
   <223> PHOSPHORYLATION: tyrosine at position 8 is phosphorylated
<400> 8
   Met Ser Lys Asp Gln Pro Asp Tyr Ala Met Tyr Ser Arg Ile Ser
   1               5                   10                  15
<210> 9
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 9
   Asp Gln Pro Asp Tyr Ala Met Tyr Ser Arg Ile Ser Ser Ser Pro
   1               5                   10                  15
<210> 10
   <211> 15
   <212> PRT
   <213> Homo sapiens
<220>
   <221> MOD_RE5
   <222> (8)..(8)
   <223> PHOSPHORYLATION: tyrosine at position 8 is phosphorylated
<400> 10
   Val Glu Arg Tyr Thr Glu Phe Tyr His Val Pro Thr His Ser Asp
   1               5                   10                  15
<210> 11
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 11
   Thr Lys Gly Pro Asn Gly Val Tyr Asp Phe Ser Gln Ala His Asn
   1               5                   10                  15
<210> 12
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 12
   Pro Leu Tyr Gly Asn Leu His Tyr Leu Gln Thr Gly Arg Leu Ser
   1               5                   10                  15
<210> 13
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 13
   Arg Asn Asp Ser Asp Pro Thr Tyr Gly Gln Gln Gly His Pro Asn
   1               5                   10                  15
<210> 14
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 14
   Pro Gln Ser Ala Pro Gln Ile Tyr Gly Pro Pro Gln Tyr Asn Ile
   1               5                   10                  15
<210> 15
   <211> 15
   <212> PRT
   <213> Homo sapiens
<220>
   <221> MOD_RES
   <222> (8) .. (8)
   <223> PHOSPHORYLATION: tyrosine at position 8 is phosphorylated,
<400> 15
   Pro Pro Gln Tyr Asn Ile Gln Tyr Ser Ser Ser Ala Ala Val Lys
   1               5                   10                  15
<210> 16
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 16
   Pro Glu Gly Asp Tyr Leu Ser Tyr Arg Glu Phe His Ser Ala Gly
   1               5                   10                  15
<210> 17
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 17
   Leu Ala Pro Gln Trp Glu Gly Tyr Asp Glu Leu Gln Thr Asp Gly
   1               5                   10                  15
<210> 18
   <211> 15
   <212> PRT
   <213> Homo sapiens
<220>
   <221> MOD_RES
   <222> (8).. (8)
   <223> PHOSPHORYLATION: tyrosine at position 8 is phosphorylated
<400> 18
   Leu Glu Gln Leu Glu Pro Asp Tyr Phe Lys Asp Met Thr Pro Thr
   1               5                   10                  15
<210> 19
   <211> 15
   <212> PRT
   <213> Homo sapiens
<220>
   <221> MOD_RES
   <222> (8).. (8)
   <223> PHOSPHORYLATION: tyrosine at position 8 is phosphorylated
<400> 19
   Tyr Gln Thr His Gln Pro Val Tyr His Lys Ile Gln Gly Asp Asp
   1               5                   10                  15
<210> 20
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 20
   Val Ala Lys Asp Lys Pro Val Tyr Asp Glu Leu Phe Tyr Thr Leu
   1               5                   10                  15
<210> 21
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 21
   Pro Val Tyr Asp Glu Leu Phe Tyr Thr Leu Ser Pro Ile Asn Gly
   1 5 10 15
<210> 22
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 22
   Asn Arg Leu Phe Gln Gln Ile Tyr Ser Asp Gly Ser Asp Glu Val
   1 5 10 15
<210> 23
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 23
   Thr Gly Ser Pro Gly Met Ile Tyr Ser Thr Arg Tyr Gly Ser Pro
   1 5 10 15
<210> 24
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 24
   Gly Leu Asp Pro Val Glu Val Tyr Glu Ser Leu Pro Glu Glu Leu
   1 5 10 15
<210> 25
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 25
   Val Leu Ala Gln Gln Gly Glu Tyr Ser Glu Ala Ile Pro Ile Leu
   1               5                   10                  15
<210> 26
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 26
   Arg His Tyr Asn Gly Glu Ala Tyr Glu Asp Asp Glu His His Pro
   1               5                   10                  15
<210> 27
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 27
   Lys Glu Gln Glu Arg Leu Ala Tyr Ile Asn Pro Asp Leu Ala Leu
   1               5                   10                  15
<210> 28
   <211> 15
   <212> PRT
   <213> Homo sapiens
<220>
   <221> MOD_RES
   <222> (8).. (8)
   <223> PHOSPHORYLATION: tyrosine at position 8 is phosphorylated
<400> 28
   Ser Gly Ala Arg Leu Gly Glu Tyr Glu Asp Val Ser Arg Val Glu
   1 5 10 15
<210> 29
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 29
   Tyr Thr Ile Ser Gln Glu Ala Tyr Asp Gln Arg Gln Asp Thr Val
   1 5 10 15
<210> 30
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 30
   Lys Ala Asp Gly Gly Ala Glu Tyr Ala Thr Tyr Gln Thr Lys Ser
   1 5 10 15
<210> 31
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 31
   Gly Gly Ala Glu Tyr Ala Thr Tyr Gln Thr Lys Ser Thr Thr Pro
   1 5 10 15
<210> 32
   <211> 15
   <212> PRT
   <213> Homo sapiens
<220>
   <221> MOD RES
   <222> (8) .. (8)
   <223> PHOSPHORYLATION: tyrosine at position 8 is phosphorylated
<400> 32
   Ala Arg Val Ile Thr Asn Gln Tyr Asn Asn Pro Ala Gly Leu Tyr
   1               5                   10                  15
<210> 33
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 33
   Tyr Asn Asn Pro Ala Gly Leu Tyr Ser Ser Glu Asn Ile Ser Asn
   1               5                   10                  15
<210> 34
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 34
   Ala Asn Tyr Asp Gly Lys Asp Tyr Asp Pro Val Ala Ala Arg Gln
   1               5                   10                  15
<210> 35
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 35
   Lys Lys Glu Asp Ala Leu Leu Tyr Gln Ser Lys Gly Tyr Asn Asp
   1 5 10 15
<210> 36
   <211> 15
   <212> PRT
   <213> Homo sapiens
<220>
   <221> MOD_RES
   <222> (8)..(8)
   <223> PHOSPHORYLATION: tyrosine at position 8 is phosphorylated,
<400> 36
   Lys Gly Tyr Asn Asp Asp Tyr Tyr Glu Glu Ser Tyr Phe Thr Thr
   1 5 10 15
<210> 37
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 37
   Asp Asp Tyr Tyr Glu Glu Ser Tyr Phe Thr Thr Arg Thr Tyr Gly
   1 5 10 15
<210> 38
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 38
   Pro Arg Phe His Thr Ser Thr Tyr Asp Leu Pro Gly Pro Glu Gly
   1 5 10 15
<210> 39
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 39
   Ala Ser Cys Arg Asn Leu Val Tyr Asp Gln Ser Pro Asn Arg Thr
   1               5                   10                  15
<210> 40
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 40
   Ala Asn Tyr Asp Gly Lys Glu Tyr Asp Pro Val Glu Ala Arg Gln
   1               5                   10                  15
<210> 41
   <211> 15
   <212> PRT
   <213> Homo sapiens
<220>
   <221> MOD_RES
   <222> (8) .. (8)
   <223> PHOSPHORYLATION: tyrosine at position 8 is phosphorylated
<400> 41
   Gly Phe Lys Val Gly Ile Asn Tyr Gln Pro Pro Thr Val Val Pro
   1               5                   10                  15
<210> 42
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 42
   Gly Ile Asp Pro Thr Gly Thr Tyr His Gly Asp Ser Asp Leu Gln
   1               5                   10                  15
<210> 43
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 43
   Tyr Ser Met Glu Ala Ala Asp Tyr Arg Glu Ala Ser Ser Gln Gln
   1               5                   10                  15
<210> 44
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 44
   Asn Leu Asn Leu Gly Arg Thr Tyr Ala Ser Gly Tyr Ala His Tyr
   1               5                   10                  15
<210> 45
   <211> 13
   <212> PRT
   <213> Homo sapiens
<220>
   <221> MOD_RES
   <222> (8)..(8)
   <223> PHOSPHORYLATION: tyrosine at position 8 is phosphorylated
<400> 45
   Tyr Ala Ser Gly Tyr Ala His Tyr Glu Glu Gln Glu Asn
   1               5                   10
<210> 46
   <211> 15
   <212> PRT
   <213> Homo sapiens
<220>
   <221> MOD_RES
   <222> (8).. (8)
   <223> PHOSPHORYLATION: tyrosine at position 8 is phosphorylated
<400> 46
   Glu Leu Asp Ser Lys Leu Asn Tyr Lys Pro Pro Pro Gln Lys Ser
   1               5                   10                  15
<210> 47
   <211> 15
   <212> PRT
   <213> Homo sapiens
<220>
   <221> MOD_RES
   <222> (8) .. (8)
   <223> PHOSPHORYLATION: tyrosine at position 8 is phosphorylated
<400> 47
   Asn Asp Asp Ser Asp Asp Ser Tyr Phe Thr Ser Ser Ser Ser Tyr
   1               5                   10                  15
<210> 48
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 48
   Gly Gln Pro Gly Asp Glu Thr Tyr Gln Asp Ile Phe Arg Asp Phe
   1               5                   10                  15
<210> 49
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 49
   Lys Gln Ala Thr Thr Asn Val Tyr Gln Val Gln Thr Gly Ser Glu
   1               5                   10                  15
<210> 50
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 50
   Gln Val Gln Thr Gly Ser Glu Tyr Thr Asp Thr Ser Asn His Ser
   1               5                   10                  15
<210> 51
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 51
   Ser Ser Lys Cys Cys Cys Ile Tyr Glu Lys Pro Arg Ala Phe Gly
   1               5                   10                  15
<210> 52
   <211> 15
   <212> PRT
   <213> Homo sapiens
<220>
   <221> MOD_RES
   <222> (8).. (8)
   <223> PHOSPHORYLATION: tyrosine at position 8 is phosphorylated
<400> 52
   Leu Arg Gly Ala Asp Ser Ala Tyr Tyr Gln Val Gly Gln Thr Gly
   1 5 10 15
<210> 53
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 53
   Ala Ser Ser Ala Gly Leu Ile Tyr Ile Asp Pro Ser Asn Leu Arg
   1 5 10 15
<210> 54
   <211> 15
   <212> PRT
   <213> Homo sapiens
<220>
   <221> MOD_RES
   <222> (8).. (8)
   <223> PHOSPHORYLATION: tyrosine at position 8 is phosphorylated
<400> 54
   Asp Thr Thr Leu Ser Asp Ser Tyr Asn Tyr Ser Gly Thr Glu Asn
   1 5 10 15
<210> 55
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 55
   Thr Leu Ser Asp Ser Tyr Asn Tyr Ser Gly Thr Glu Asn Leu Lys
   1 5 10 15
<210> 56
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 56
   Val Glu Ser Leu Glu Ser Leu Tyr Phe Thr Pro Ile Pro Ala Arg
   1               5                   10                  15
<210> 57
   <211> 15
   <212> PRT
   <213> Homo sapiens
<220>
   <221> MOD_RES
   <222> (8) .. (8)
   <223> PHOSPHORYLATION: tyrosine at position 8 is phosphorylated
<400> 57
   Trp Ser Lys Asp Ile Asn Ala Tyr Asn Cys Glu Glu Pro Thr Glu
   1               5                   10                  15
<210> 58
   <211> 13
   <212> PRT
   <213> Homo sapiens
<220>
   <221> MOD_RES
   <222> (8)..(8)
   <223> PHOSPHORYLATION: tyrosine at position 8 is phosphorylated
<400> 58
   Asp Val Gln Lys Ser Lys Glu Tyr Phe Ser Lys Gln Lys
   1               5                   10
<210> 59
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 59
   Gly Gln Arg Ala Gln Glu Asn Tyr Glu Gly Ser Glu Glu Val Ser
   1 5 10 15
<210> 60
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 60
   Leu Cys Arg Ala Asp Gln Gln Tyr Glu Cys Val Ala Glu Ile Gly
   1 5 10 15
<210> 61
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 61
   Ala Glu Ile Gly Glu Gly Ala Tyr Gly Lys Val Phe Lys Ala Arg
   1 5 10 15
<210> 62
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 62
   Ser Asn Asp Gln Asp Gly Glu Tyr Cys Pro Glu Val Lys Leu Lys
   1 5 10 15
<210> 63
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 63
   Leu Lys Ala Asp Gly Leu Ile Tyr Cys Leu Lys Glu Ala Cys Pro
   1               5                   10                  15
<210> 64
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 64
   Ser Gly Ser Trp Ala Ala Ile Tyr Gln Asp Ile Arg His Glu Ala
   1               5                   10                  15
<210> 65
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 65
   Ala Asn Lys Glu Val Leu Asp Tyr Ser Thr Pro Thr Thr Asn Gly
   1               5                   10                  15
<210> 66
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 66
   Ile Cys His Asn Gln Asp Gly Tyr Ser Tyr Pro Ser Arg Gln Met
   1               5                   10                  15
<210> 67
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 67
   Phe Gly Gly Glu Pro Leu Ser Tyr Thr Arg Phe Ser Leu Ala Arg
   1               5                   10                  15
<210> 68
   <211> 15
   <212> PRT
   <213> Homo sapiens
<220>
   <221> MOD_RES
   <222> (8).. (8)
   <223> PHOSPHORYLATION: tyrosine at position 8 is phosphorylated
<400> 68
   Gly Met Asp Asn Gln Gly Gly Tyr Gly Ser Val Gly Arg Met Gly
   1               5                   10                  15
<210> 69
   <211> 15
   <212> PRT
   <213> Homo sapiens
<220>
   <221> MOD_RES
   <222> (8) .. (8)
   <223> PHOSPHORYLATION: tyrosine at position 8 is phosphorylated
<400> 69
   Ala Val Pro Lys Glu Asp Ile Tyr Ser Gly Gly Gly Gly Gly Gly
   1               5                   10                  15
<210> 70
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 70
   Gly Tyr Gly Gly Tyr Glu Glu Tyr Ser Gly Leu Ser Asp Gly Tyr
   1               5                   10                  15
<210> 71
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 71
   Gly Tyr Gly Gly Tyr Asp Asp Tyr Asn Gly Tyr Asn Asp Gly Tyr
   1               5                   10                  15
<210> 72
   <211> 15
   <212> PRT
   <213> Homo sapiens
<220>
   <221> MOD RES
   <222> (8)..(8)
   <223> PHOSPHORYLATION: tyrosine at position 8 is phosphorylated
<400> 72
   Val Ala Asp Leu Thr Glu Gln Tyr Asn Glu Gln Tyr Gly Ala Val
   1               5                   10                  15
<210> 73
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 73
   Thr Glu Gln Tyr Asn Glu Gln Tyr Gly Ala Val Arg Thr Pro Tyr
   1               5                   10                  15
<210> 74
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 74
   Lys Gln Ser Asp Asp Glu Val Tyr Ala Pro Gly Leu Asp Ile Glu
   1               5                   10                  15
<210> 75
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 75
   Met Pro Lys Phe Tyr Cys Asp Tyr Cys Asp Thr Tyr Leu Thr His
   1               5                   10                  15
<210> 76
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 76
   Tyr Cys Asp Tyr Cys Asp Thr Tyr Leu Thr His Asp Ser Pro Ser
   1               5                   10                  15
<210> 77
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 77
   Ser Leu Lys Tyr Glu Asn Asp Tyr Pro Ser Val Ile Leu Arg Asp
   1               5                   10                  15
<210> 78
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 78
   Thr Asp Thr Leu Gln Asn Asp Tyr Phe Ala Ile Lys Gln Glu Val
   1               5                   10                  15
<210> 79
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 79
   Thr Ser Leu Asn Ser Asn Asp Tyr Gly Tyr Gly Gly Gln Lys Arg
   1               5                   10                  15
<210> 80
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 80
   Ala Asn Ile Gly Glu Asp Thr Tyr Asp Ile Val Ile Pro Val Lys
   1 5 10 15
<210> 81
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 81
   Pro Ser Ser Asp Asn Ser Leu Tyr Thr Asn Ala Lys Gly Ser Thr
   1 5 10 15
<210> 82
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 82
   Glu Ala Val Val Glu Glu Asp Tyr Asn Glu Asn Ala Lys Asn Gly
   1 5 10 15
<210> 83
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 83
   Glu Ala Val Val Glu Glu Asp Tyr Asn Glu Asn Ala Lys Asn Gly
   1 5 10 15
<210> 84
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 84
   Gly Val Val Gly Gly Glu Asp Tyr Glu Glu Val Asp Arg Tyr Ser
   1 5 10 15
<210> 85
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 85
   Thr Thr Asp Phe Pro Gly Asn Tyr Ser Gly Tyr Asp Asp Ala Trp
   1 5 10 15
<210> 86
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 86
   Lys Leu Val Val Glu Glu Tyr Tyr Glu Val Leu Asp His Cys Ser
   1 5 10 15
<210> 87
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 87
   Val Ile Asp Asp Glu Ser Asp Tyr Phe Ala Ser Asp Ser Asn Gln
   1               5                   10                  15
<210> 88
   <211> 15
   <212> PRT
   <213> Homo sapiens
<220>
   <221> MOD_RES
   <222> (8).. (8)
   <223> PHOSPHORYLATION: tyrosine at position 8 is phosphorylated
<400> 88
   Ile Gln Pro Gly Glu Gln Lys Tyr Glu Tyr Lys Ser Asp Gln Trp
   1               5                   10                  15
<210> 89
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 89
   Ser Leu Lys Glu Ala Leu Thr Tyr Asp Gly Ala Leu Leu Gly Asp
   1               5                   10                  15
<210> 90
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 90
   Lys Val Glu Arg Ala Asp Gly Tyr Glu Pro Pro Val Gln Glu Ser
   1               5                   10                  15
<210> 91
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 91
   Ala Asp Ile Asp Glu Gly Leu Tyr Ser Arg Gln Leu Tyr Val Leu
   1               5                   10                  15
<210> 92
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 92
   Glu Pro Glu Glu Asp Leu Glu Tyr Phe Glu Cys Ser Asn Val Pro
   1               5                   10                  15
<210> 93
   <211> 15
   <212> PRT
   <213> Homo sapiens
<220>
   <221> MOD_RES
   <222> (8)..(8)
   <223> PHOSPHORYLATION: tyrosine at position 8 is phosphorylated
<400> 93
   Thr Arg Glu Pro Pro Pro Ala Tyr Glu Pro Pro Ala Pro Ala Pro
   1               5                   10                  15
<210> 94
   <211> 13
   <212> PRT
   <213> Homo sapiens
<220>
   <221> MOD_RES
   <222> (8)..(8)
   <223> PHOSPHORYLATION: tyrosine at position 8 is phosphorylated
<400> 94
   Ala Asp Glu Glu Glu Asp Glu Tyr Ser Gly Gly Leu Cys
   1               5                   10
<210> 95
   <211> 15
   <212> PRT
   <213> Homo sapiens
<220>
   <221> MOD_RES
   <222> (8)..(8)
   <223> PHOSPHORYLATION: tyrosine located at position 8 is phosphorylate d
<400> 95
   Pro Gln Asp Leu Thr Asp Ala Tyr Gly Pro Pro Ser Asn Phe Leu
   1               5                   10                  15